# EUROPEAN PATENT SPECIFICATION

(11) **EP 2 498 853 B1**
(45) Date of publication and mention of the grant of the patent: **20.06.2018**
(21) Application number: 10830251.4
(22) Date of filing: 12.11.2010
(51) Int. Cl.: A61M 16/06, A61M 16/08

(54) **PATIENT INTERFACE AND ASPECTS THEREOF**
PATIENTENSCHNITTSTELLE UND ASPEKTE DAVON
INTERFACE PATIENT ET SES ASPECTS

(30) Priority: 12.11.2009 US 260590 P; 10.05.2010 WO PCT/IB2010/052061; 23.08.2010 US 376067 P
(43) Date of publication of application: 19.09.2012
(73) Proprietor: Fisher & Paykel Healthcare Limited, East Tamaki, Auckland 2013 (NZ)
(72) Inventor: SALMON, Andrew, Paul, Maxwell, Epsom, Auckland 1023 (NZ); SIEW, Silas, Sao, Jin, Flat Bush, Auckland 2016 (NZ); HUANG, Wen, Dong, Pakuranga, Auckland 2010 (NZ); ALLAN, Olivia, Marie, Meadowbank, Auckland 1072 (NZ); MCLAREN, Mark, Pakuranga, Auckland 2010 (NZ); PRENTICE, Craig, Robert, Botany Downs North, Auckland 2010 (NZ); GARDIOLA, Arvin, San, Jose, Bucklands Beach, Auckland 2012 (NZ); MACULEY, Alastair, Edwin, Mt Eden, Auckland 1024 (NZ)
(74) Representative: Hoarton, Lloyd Douglas Charles
(86) International application number: PCT/NZ2010/000225
(87) International publication number: WO 2011/059346

(56) References cited:
- EP-A2- 2 022 528
- WO-A1-2009/052560
- WO-A1-2009/139647
- WO-A1-2010/139014
- US-A1- 2002 059 935
- US-A1- 2005 028 822
- US-A1- 2006 054 169
- US-A1- 2006 266 361
- US-A1- 2007 089 749
- US-A1- 2009 120 442

## Description

### FIELD OF THE INVENTION

The present invention relates to patient interfaces for delivering breathing gases to a patient and aspects of patient interfaces.

### SUMMARY OF THE PRIOR ART

The present invention relates to patient interfaces for delivering breathing gases to a patient. The invention will be particularly described with reference to patient interfaces for delivering PAP therapy to a patient, for example, to a patient suffering from obstructive sleep apnea (OSA). However, the patient interface could be used for other treatments. Furthermore, aspects of the patient interface could be combined with aspects of other patient interfaces for use in PAP therapy or for use in other therapy.

Desirable traits for patient interfaces used in PAP therapy include lightweight, comfortable, intuitive to use, good seal and stable and secure during use.

US 2009/120442 discloses a patient interface that includes a body member, a partition, a nasal seal portion, and a port. The partition is integral with the body member, and separates an interior of the member into a first chamber configured to receive a nose of a patient and a second chamber. The nasal seal portion is integral with the partition, and projects therefrom into the first chamber. The nasal seal portion includes a seal surface and a gas passage therethrough. The seal surface is configured to conform with a lower portion of the nose that is surrounding nasal passages of the patient, without entering the nasal passages of the patient, when the patient interface is mounted operatively on the face of the patient. The port integral with the member is in fluid communication with the second chamber for delivery of gas to the patient via the gas passage in the nasal seal portion.

In this specification where reference has been made to patent specifications, other external documents, or other sources of information, this is generally for the purpose of providing a context for discussing the features of the invention. Unless specifically stated otherwise, reference to such external documents is not to be construed as an admission that such documents, or such sources of information, in any jurisdiction, are prior art, or form part of the common general knowledge in the art.

### SUMMARY OF THE INVENTION

The invention is defined by the appended claims.

An object of the present invention is to provide a patient interface, or aspects of a patient interface which will at least provide the public with a useful choice.

The present invention provides a patient interface as claimed.

The term "comprising" is used in the specification and claims, means "consisting at least in part of". When interpreting a statement in this specification and claims that includes "comprising", features other than that or those prefaced by the term may also be present. Related terms such as "comprise" and "comprises" are to be interpreted in the same manner.

In this specification where reference has been made to patent specifications, other external documents, or other sources of information, this is generally for the purpose of providing a context for discussing the features of the invention. Unless specifically stated otherwise, reference to such external documents is not to be construed as an admission that such documents, or such sources of information, in any jurisdiction, are prior art, or form part of the common general knowledge in the art.

### BRIEF DESCRIPTION OF THE DRAWINGS

Preferred forms of the present invention will be described with reference to the accompanying drawings.
**Figure 1** is a perspective view of a person wearing a patient interface.
**Figure 2** is a perspective view of the patient interface of Figure 1 without the patient.
**Figure 3** is an exploded view illustrating the components making up the interface of Figure 2.
**Figures 4A** to **4C** illustrate, from different angles, a seal component of the patient interface of Figure 2. Figure 4A shows the seal component from an outward facing side, Figure 4B shows the seal from a patient-facing side and Figure 4C shows a side view of the seal.
**Figure 5A** is an exploded view of the seal and mask frame showing how they can be brought together to be assembled.
**Figure 5B** is a side view of the interface of Figure 2, partially disassembled to show the connection of an elbow to the mask frame.
**Figure 5C** is a front perspective view of the interface of Figure 2 illustrating assembly of the elbow with the mask frame, with a gas washout vent present in the elbow.

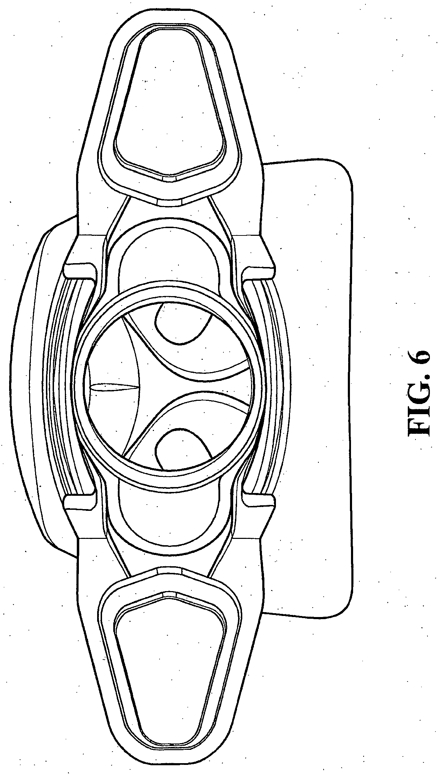

**Figure 6** is a front view of the assembled seal and mask frame.
**Figure 7A** is a top view of the assembled seal and mask frame.
**Figure 7B** is a side view of the seal and frame of Figure 7A, unsectioned.
**Figure 7C** is a view of the patient side of the seal of Figures 4A and 4C.
**Figure 7D** is a side elevation of the seal of Figure 7C, sectioned through line EE.
**Figure 8** is a side view of the seal and mask frame of Figure 7A taken through line DD.
**Figure 9A** is a top view of the seal of Figure 7C, sectioned through line FF of Figure 7D.
**Figure 9B** is a top view of the seal of Figure 7C, sectioned through line GG of Figure 7D.
**Figures 10A to 10C** are perspective views illustrating a patient interface incorporating a number of inventions described in this specification. Figure 10A is a front view, Figure 10B is a profile view, and Figure 10C is a back view.
**Figures 11A to 11H** are views of the soft seal component of the interface of Figures 10A to 10C. Figure 11A is a top view. Figure 11B is a front view. Figure 11C is a rear view taken from a position directly facing the open end of a nasal locator. Figure 11D is a cross section through section CC in Figure 11C. Figure 11E is a cross section through line DD in Figure 11C. Figure 11F is a cross section through line EE in Figure 11E. Figure 11G is a cross section through line GG in Figure 11E. Figure 11H is a cross section through line HH in Figure 11E.
**Figures 12A to 12H** are views of a frame assembly of the interface of Figures 10A to 10C. Figure 12A is a front perspective view of the frame assembly. Figure 12B is a rear perspective view of the frame assembly. Figure 12C is a side profile of the frame assembly. Figure 12D is an assembly view of two components of the frame assembly. Figure 12E is a back view of the frame assembly. Figure 12F is a front view of the frame assembly. Figure 12G is a cross sectional side view taken through line AA of Figure 12F. Figure 12H is a cross sectional top view, taken through line BB in Figure 12F.
**Figure 12I** is a rear view of an alternative frame assembly having a different channel path for securing the lip of the seal.
**Figure 13** shows a further embodiment of an interface with an inflatable seal. The interface includes a seal body, frame, tubing and head strap.
**Figure 14** shows the seal body of the interface of Figure 13.
**Figure 15** is a perspective view of the frame and seal body of the interface of Figure 13.
**Figure 16** is a front view of the frame and seal body of the interface of Figure 13.
**Figure 17** is a side view of the frame and seal body of the interface of Figure 13.
**Figure 18** is a cross-section of the frame and seal body through BB in Figure 17.
**Figure 19** is an alternative cross-sectional view of the seal body.
**Figure 20** is a cross-section of the seal body through AA of Figure 16.
**Figure 21** is an alternative embodiment of a seal body of the interface of the present invention.
**Figure 22** is yet a further embodiment of a seal body of an interface of the present invention.
**Figure 23** is another embodiment of a seal body of an interface of the present invention.
**Figure 24** shows a first embodiment of a head strap that may be used with an interface of the present invention.
**Figures 24a and 24b** show two alternative cross-sections of the head strap of Figure 24.
**Figure 25** shows a second embodiment of a head strap that may be used with an interface of the present invention.
**Figures 25a and 25b** show two alternative cross-sections of the head strap of Figure 25.
**Figure 26** shows a third embodiment of a head strap that may be used with an interface of the present invention.
**Figure 27** shows a fourth embodiment of a head strap that may be used with an interface of the present invention.
**Figure 27a** shows the extendible portion of the head strap of Figure 27 in extended and contracted conditions.
**Figure 28** shows a fifth embodiment of a head strap that may be used with an interface of the present invention.
**Figure 29** shows a sixth embodiment of a head strap that may be used with an interface of the present invention.
**Figure 30** shows a first embodiment of the connection between a seal body and frame of the interface of the present invention.
**Figure 31** shows a second embodiment of the connection between a seal body and frame of the interface of the present invention.
**Figure 32** shows a third embodiment of the connection between a seal body and frame of the interface of the present invention.
**Figure 33** shows a forth embodiment of the connection between a seal body and frame of the interface of the present invention.
**Figure 34** shows a first embodiment of a brace that may be used with the interface of the present invention to fix tubing connected to the interface to the user.
**Figure 35** shows a second embodiment of a brace that may be used with the interface of the present invention to fix tubing connected to the interface to the user.
**Figure 36** shows a third embodiment of a brace that may be used with the interface of the present invention to fix tubing connected to the interface to the user.
**Figure 37** shows a fourth embodiment of a brace that may be used with the interface of the present invention to fix tubing connected to the interface to the user.
**Figure 38** shows a fifth embodiment of a brace that may be used with the interface of the present invention to fix tubing connected to the interface to the user.
**Figure 39** shows a sixth embodiment of a brace that may be used with the interface of the present invention to fix tubing connected to the interface to the user.
**Figure 40** shows a seventh embodiment of a brace that may be used with the interface of the present invention to fix tubing connected to the interface to the user.
**Figure 41** is a perspective view of a supporting collar.
**Figure 42** is a perspective view of a patient wearing the supporting collar of Figure 41.
**Figure 43** is a perspective view of the collar of Figure 41 from a different angle.
**Figure 44** is a front view of a patient wearing the collar of Figure 41.
**Figure 45** is a top view of the collar of Figure 41.
**Figure 46** is a top view of a portion of the collar of Figure 41 illustrating a dome fastener connector.
**Figure 47** is a top view of a portion of the collar including an alternative fastener.
**Figure 48** is a top view of a portion of the collar including an alternative fastener.
**Figure 49** is a top view of a portion of the collar including a further alternative fastener.
**Figure 50A** is a side view of a portion of the collar including a securing clip in an engaged configuration.
**Figure 50B** is a side view of the portion of the collar from Figure 50A in a disengaged condition.
**Figure 51A** is a side view of a portion of the collar including a securing clip according to an alternative embodiment in a disengaged condition.
**Figure 51B** is a side view of the portion of the collar of Figure 51A in an engaged condition.
**Figure 52** is a perspective view of a tether depending from a portion of the collar, with the tether including a quick disconnect connector.
**Figure 53** is a perspective view illustrating a tether depending from the collar, the tether including a quick disconnect connector which can also swivel.
**Figure 54A** is a top view of a tether similar to the tether of Figure 51B, but of minimal length.
**Figure 54B** is a top view of the tether of Figure 54A with the quick release connector disengaged.
**Figure 54C** is a side view of the tether of Figure 54A.
**Figure 55** is an exploded view illustrating connection of a ring of the tether from the supporting collar to a conduit.
**Figure 56** is a perspective view of an interface including an alternative arrangement for supporting the conduit from the patient.
**Figure 57** is a perspective view of a patient wearing an interface including a further alternative arrangement for supporting the conduit on the patient.
**Figure 58** is a side elevation of a frame and seal, the frame incorporating depending lip stabilisers in accordance with a further invention herein.
**Figure 59** is a top view of the frame and seal of Figure 58.
**Figure 60** is a front view of the frame and seal of Figure 58.
**Figure 61** is a perspective view of an interface incorporating the frame and seal of Figure 58.
**Figure 62** is a graph of tested extension forces against extension of sample strap materials.
**Figures 63A to 63C** illustrate aspects of a preferred headgear of the interface of Figures 10A to 10C. Figure 63A is a perspective view of a portion of a headgear, including an end of a headstrap and a connector. Figure 63B is a top view of the strap and connector. Figure 63C is a view showing both (in cross-section) the connector and the socket of the frame assembly which receives the connector in use.

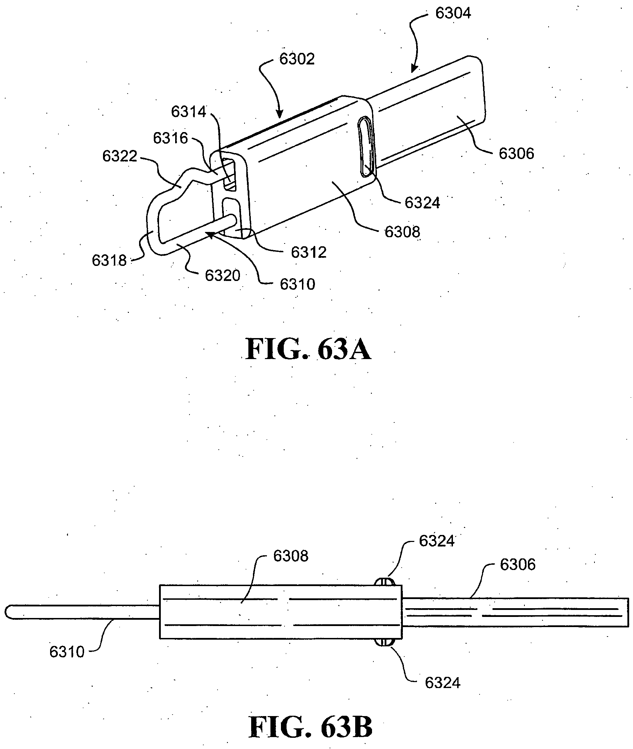

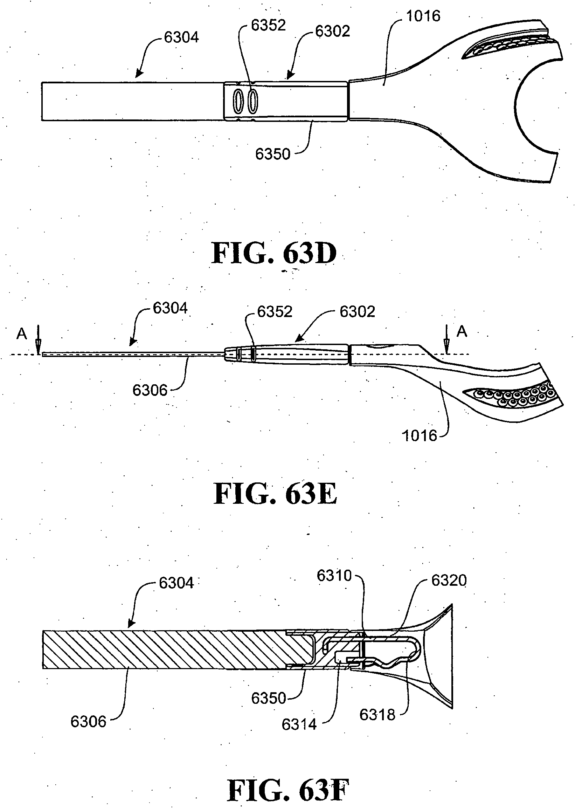

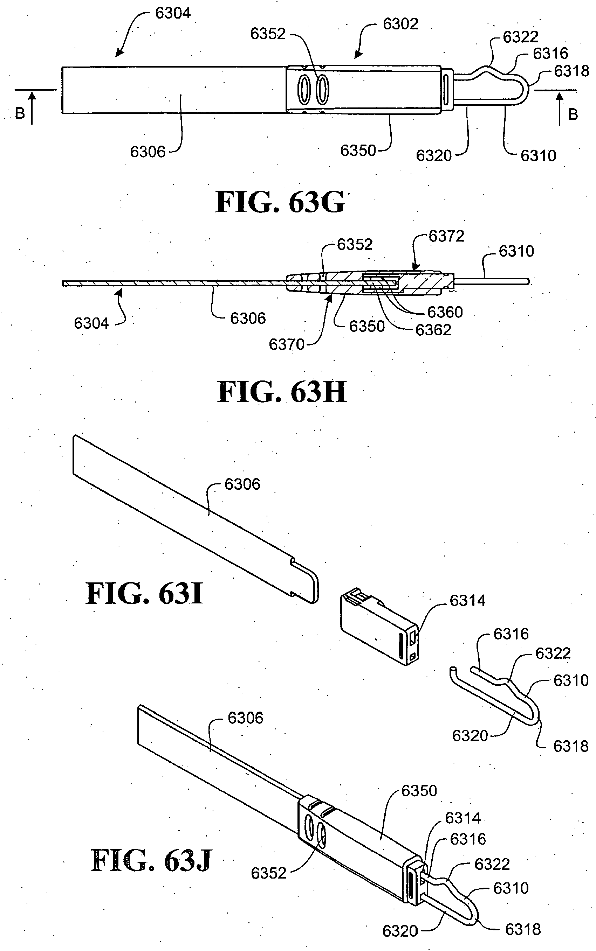

**Figures 63D to 63I** illustrate another preferred headgear. Figure 63D shows a portion of the headgear, connector and a portion of the frame assembly to which it connects. Figure 63E is a top view of the assembly of Figure 63D. Figure 63F is a sectional view through line AA of Figure 63E. Figure 63G is a side elevation of the strap portion and connector of Figure 63G. Figure 63H is a cross-section through line BB of the connector and strap portion of Figure 63G. Figure 63I is an assembly drawing of an exploded view of the strap portion and connector of Figure 63G. Figure 63J is a perspective view of the strap portion and connector of Figure 63B.
**Figure 64** is a graph of tested extension force against extension of another sample strap material.
**Figure 65** is a diagram illustrating a test apparatus for measuring the flexibility of a breathing tube.

### DETAILED DESCRIPTION

The inventions herein relate, especially but not exclusively, to an interface that includes an inflatable nasal seal having a supple wall structure. The inflatable seal has a pair of locating protrusions that engage in the nostrils of the user. The locating protrusions supply gases flow to the user from inside the seal. The patient side of the seal is so supple, and of sufficient dimension and shape, that when the inflated seal is pressed against the face of a user, with the locating protrusions engaged in the nostrils of the user, the seal conforms to the surfaces of the user's face (particularly the sides of the nose and the upper lip) and provides a seal. An outwardly facing wall of the seal is more rigid, and supports the inner wall of the seal in a position wrapped around the wearer's nose.

The seal is formed from a material having sufficient elasticity and yield strength that the combination renders the envelope supple. The supple portion is capable of repeated drastic deformations without failure. Possible materials include latex, vinyl, silicone and polyurethane. Typically wall thickness of the supple portions of the seal would be below 0.5mm and could be lower than 0.2mm

The seal body includes a pair of nasal locators protruding from the patient facing wall. Preferably the nasal locators are formed integral with the seal. Each nasal locator includes an outlet aperture for supplying gas from inside the envelope to a user wearing the interface.

The seal body includes an inlet opening, approximately opposite the nasal locators.

A substantial extent of the seal body or envelope is supple. A region adjacent and including the nasal locators and a region adjacent and including the inlet opening are much stiffer. These areas hold the overall shape of the seal and can be of any suitable stiffness. These areas may be formed of a stiffer material, or formed thicker in the same material as the rest of the envelope.

The seal is supported by a mask body or frame. The inlet opening of the seal is fitted to the frame, or directly to a conduit extending through the frame.

The frame is preferably a minimal design to provide little visual obstruction, allowing a clear field of view and allowing the user to wear glasses while wearing the interface.

The frame may be formed by injection moulding, for example from an elastomeric material such as silicone or polyurethane. Alternatively, more rigid materials such as polycarbonate, or polyester, polystyrene or nylon may be used.

The preferred frame includes connection points for connecting straps to the frame. The strap attachment points provide for anchoring the straps.

In other forms the nasal seal body may include integral strap attachment points. These attachments may be connection elements on the surface of the envelope. However, they could be integral straps or wings formed in the envelope that extend out either side of the envelope.

The interface is intended to be supported by a single strap passing around the back of the head. The strap may be formed from an elastic or elastomeric material. For example suitable strap materials may include a woven elastic strip or a narrow strip of foam and fabric, such as Breathoprene™. The strap extending around the back of the head provides pressure on the mask and helps keep the seal against the user's face.

A preferred strap according to one or more inventions herein is described later in this specification.

A flexible tube extends from the frame. The flexible tube delivers breathable gas. The distal end of the flexible tube connects to the main CPAP delivery tube.

A connector may connect the tube and the frame. The connection mechanism may be any suitable connection. This could include a snap fit, hooks into the silicone, keyhole inserts, over moulding, insert moulding, screw attachments or gluing, or any combination.

The connector may include a limited flow outlet (or bias flow outlet) for providing gas washout from the interface. The outlet may be in the form of a collection of small apertures in the connector. Internally the connector may include a funnel or extension leading from the vent into the mouth of the envelope. Alternatively, the connector may not include a bias flow vent.

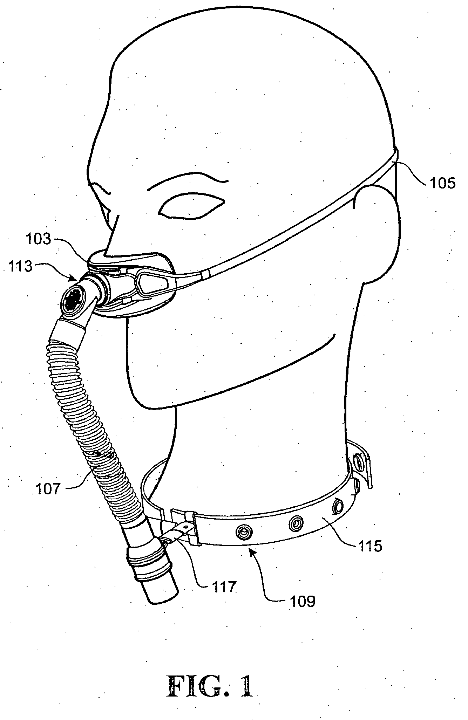

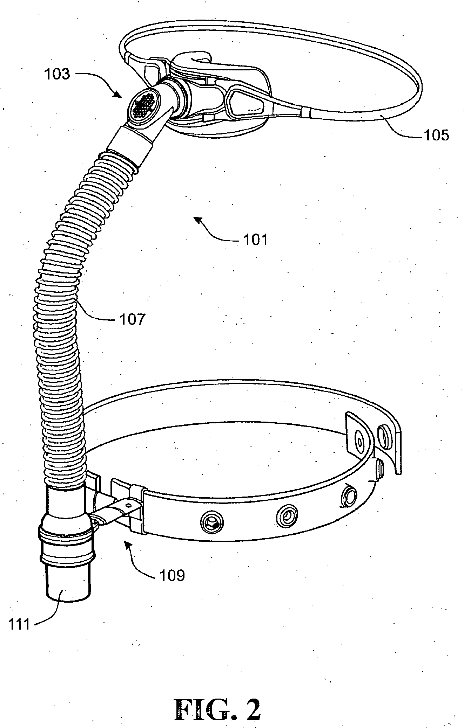

Figures 1 and 2 illustrate an example patient interface incorporating inventions disclosed in this application. For clarity, the patient interface is shown separate from the patient in Figure 2 and as worn by a patient in Figure 1.

The patient interface 101 broadly includes a mask 103, a strap 105 for securing the mask to the patient, a flexible supply conduit 107 connecting to the mask 103 and a conduit support structure 109 which attaches to a patient and supports the weight of conduit portion 107 and of any connected conduit supplying gases to the inlet end 111 of conduit portion 107.

Particular aspects of this patient interface, and variations on each aspect, will be discussed with reference to other Figures. An interface may incorporate some aspects but not other aspects. For example, an interface might incorporate aspects of the mask while using a different arrangement for securing the mask to the user. An interface might include a different mask while using inventive aspects of the strap to secure that mask to the user. An interface may incorporate aspects of the mask but not make use of a similar, or any, structure for supporting the weight of the conduit from the body of the patient. All of these variations are considered within the scope of this application.

Referring to Figure 1, the mask 103 fits over the nostrils of the patient and includes lateral portions which curve around either side of the nose. These lateral portions provide for forming a perimeter seal on the outwardly facing surfaces of the flanks of the nose. The strap 105 passes around the user's head in a simple loop above the user's ears.

The conduit portion 107 depends from a central connection 113 at the front of mask 103. The central connection 113 is preferably a swivelling elbow so that the path of the conduit relative to the positioning of the mask on the face of the patient can adapt to the sleeping position of the patient. The swivelling elbow may be in the form of a ball joint so that the elbow can pivot about axes parallel to and perpendicular to its connection with the mask.

The illustrated conduit support 109 comprises a collar 115 connected around the neck of the user. A tether 117 connects between the collar and the conduit 107.

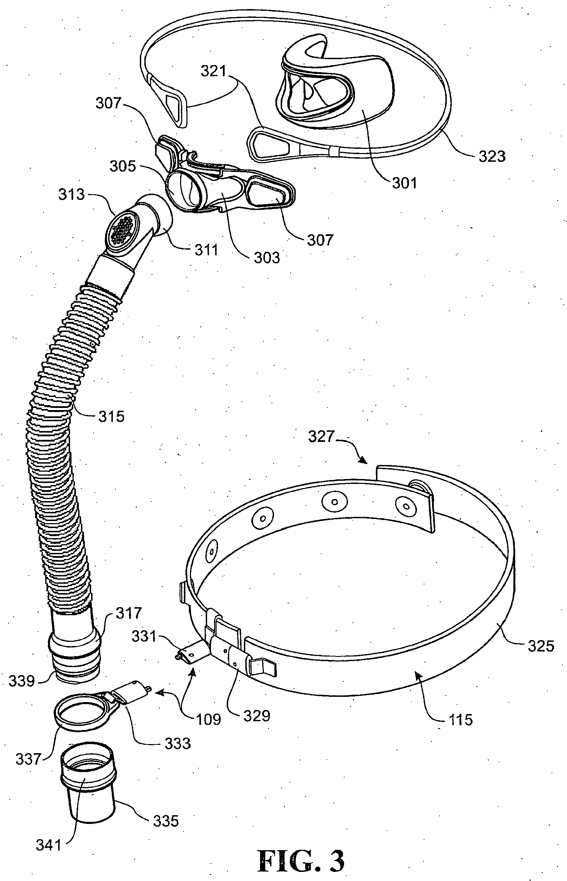

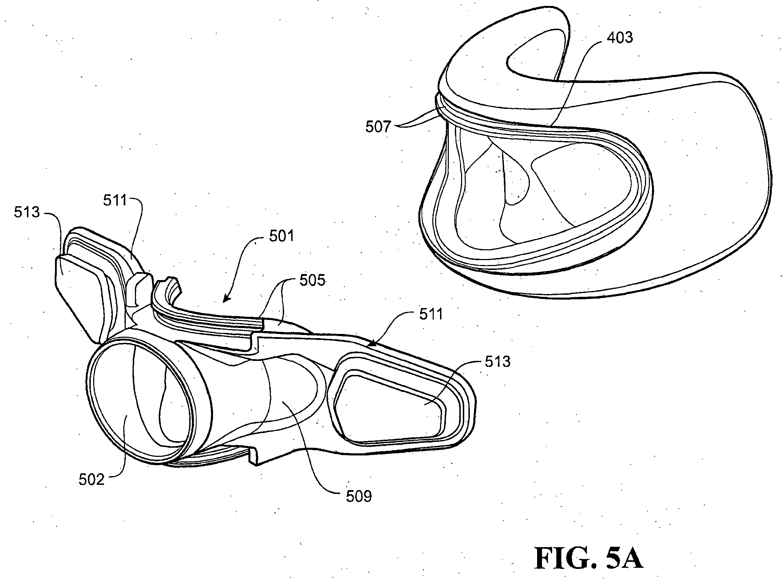

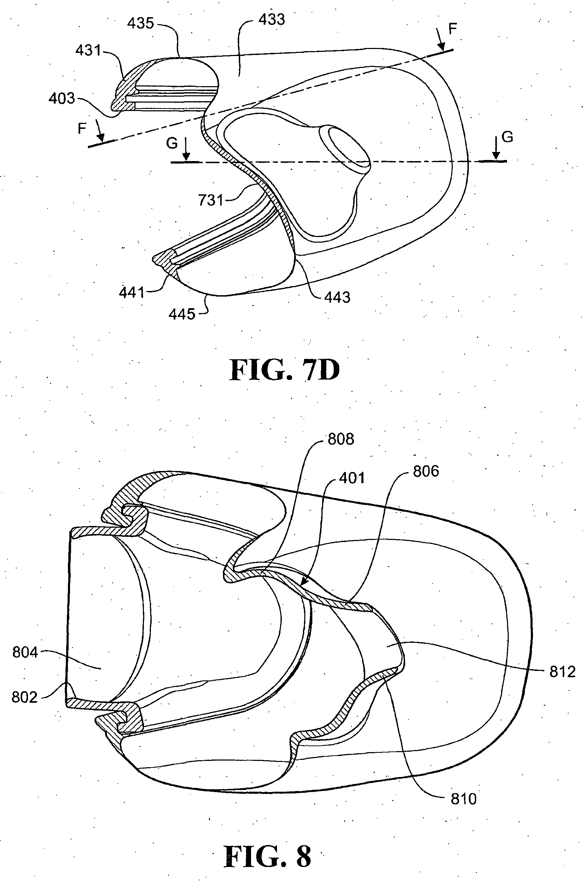

The component parts of this exemplary interface are illustrated in Figure 3. The mask 103 includes a seal 301 and a body or frame 303. The seal and body are illustrated in more detail in Figure 5A. Their engagement will be described in more detail with reference to Figure 5A and Figure 8.

The body 303 includes a socket 305 and connector portions 307. Socket 305 receives an engagement portion 311 of elbow 333. Elbow 333 is connected to the end of a length of flexible tubing 315. The other end of flexible tubing 315 is terminated with a cuff 317. Connector portions 307 of mask body 303 are for engagement with connector portions 321 of the head strap 105. The head strap 105 includes a single length 323 of stretchable material. Connector portions 321 are provided at either end of the length 323.

The collar 115 includes a band 325 of a material intended to be comfortable when worn during periods of sleep. The band includes a first adjustable connection 327 and a second non-adjustable connection 329. At the adjustable connection 327, free ends of the band overlap, and the degree of this overlap may be varied to a desired amount and fixed at this desired amount. At the non-adjustable connection 329, free ends of the band may simply be secured or released. Once the adjustable connection 327 has been adjusted, the collar may be opened for fitting to the patient or removal from the client and secured after fitting to the client, by disconnecting or connecting the non-adjustable connection 329. The non-adjustable connection 329 may be a quick release connector that disconnects with applied tension within predetermined range. This ensures that the collar will release without injuring the patient if any adverse situation arises.

The tether 109 includes a first portion 331 attached to the collar and a second portion 333 which attaches to the conduit. These portions are preferably releasably engaged by another quick release connector, which preferably disconnects on application of a tension within a predetermined range.

The tether portion 333 connects to conduit 315. The tether portion 333 includes a portion of the quick release connector and a fitting 337 that engages the conduit. The fitting 337 may be an open clip to engage a corrugation of the conduit, or a recess of the cuff 317, or to engage around the general cylindrical shape of the conduit, or to a cylindrical portion of the cuff. Alternatively, and as illustrated, the portion 337 may comprise a ring that fits around a portion of the conduit, or a portion of the cuff. In the illustrated embodiment, the ring 337 is captive between the cuff 317 and a connector portion of a swivel 335. The ring fits over a portion 339 of cuff 317 and is held captive by an end portion 341 of the swivel 335, which has a larger diameter than the internal diameter of the ring.

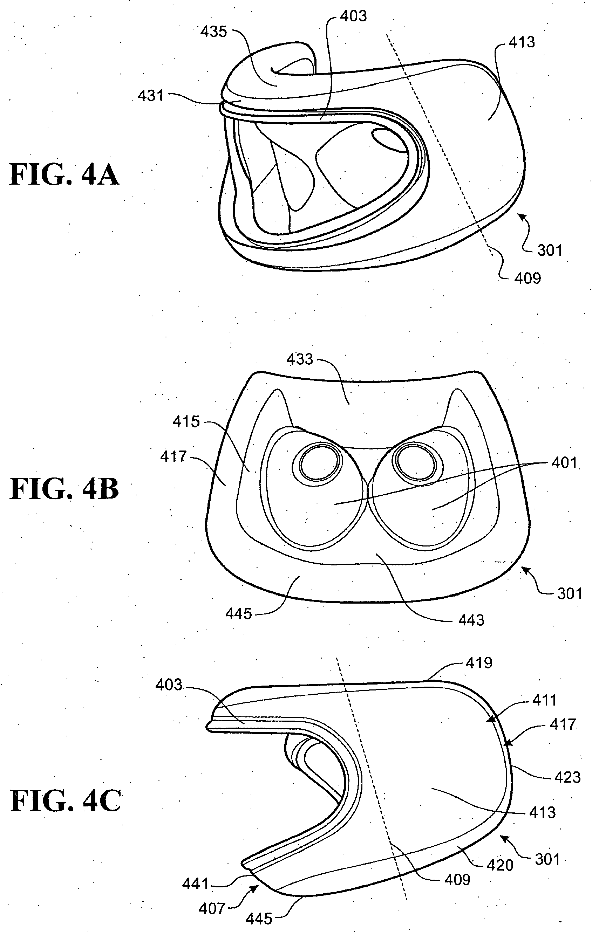

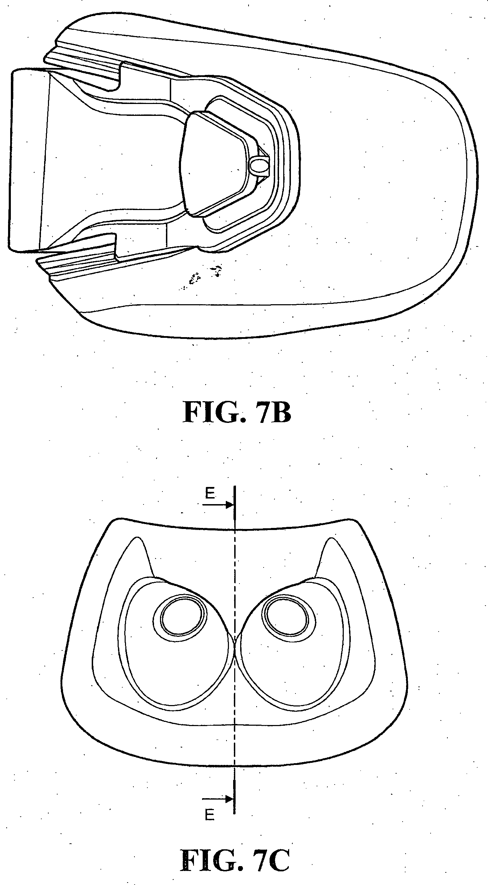

The external form of a preferred seal is illustrated in Figures 4A to 4C. The seal 301 includes a patient-facing side, broadly illustrated in Figure 4B, and an outward-facing side, broadly illustrated in Figure 4A. A pair of nasal locators 401 protrude from the patient-facing side. Broadly speaking, the wall of the seal forming the patient-facing side is very supple with the exception of the nasal locators, the area immediately adjacent the nasal locators, or both. Variations in the suppleness will be described in more detail with reference to the cross sections illustrated in Figure 7D, Figure 8 and Figure 9A and 9B.

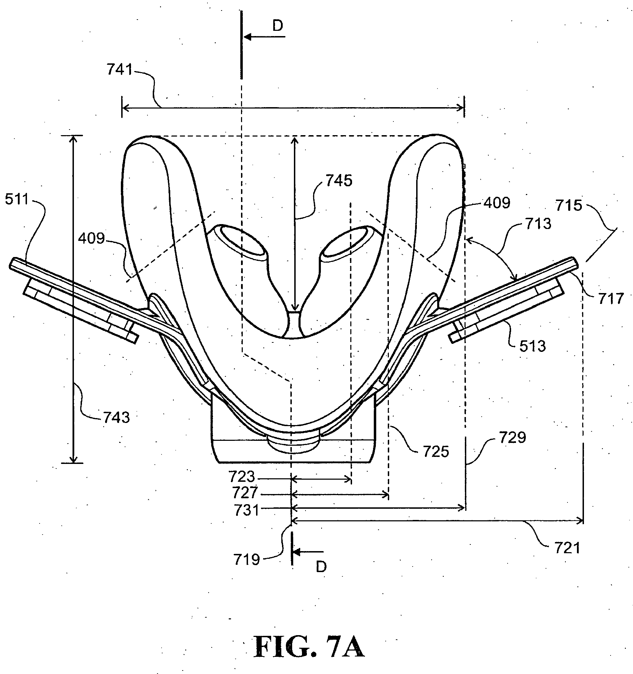

In overall form, the seal has a central portion including nasal locators on the patient-facing side and an opening 403 on the outward-facing side. The extent of this central portion 407 is broadly indicated by broken line 409 in Figures 4C and 4A. For clarity, broken line 409 is also included in Figure 7A which comprises a top view of the mask portion of the interface.

Lateral or side portions 411 extend from the central portion 407. Each side portion includes an outward face 413 and an inward face 415 and a peripheral edge portion 417 that joins the inward face portion and the outward face portion. The peripheral edge portion 417 extends around a top edge 419, an end edge 423 and a lower edge 420. Accordingly, considered from inside the seal, the side portions 411 resemble a pocket.

Each side portion is quite extensive. Preferably, the side portion extends greater than 10mm (most preferably greater than 20mm) or at least a distance 70% of the distance separating the centres of the nostril locators 401 beyond the base of each nostril locator.

At least the inside wall 415 and the perimeter wall 417 of each side portion are very supple, so that they can conform to contours of the user's face, and in particular, to contours of the outside of the sides of the user's nose. At least portions of the outside facing wall 413 of the side portion are also supple, but may be progressively less supple moving toward the central portion 407.

The central portion 407 of the seal includes opening 403 to pass a gases flow to and from the mask body 303. The opening 403 may include features such as lips and/or channels to engage with features such as channels and/or lips on the body 303. The opening 403 may be formed with clip portions, or clip portions may be attached to or over-moulded to the perimeter of the opening 403 to facilitate engagement with the frame 303. Typically, the opening 403 will be substantially thicker and more rigid than the supple sealing portions of the seal 301. The opening 403 should be at least the size of the interior cross section of the supply conduit 315. Preferably, and as illustrated, the opening 403 is commensurate with the extent of the body 303 of the mask, this extent being commensurate with the general width of the interface and approximately with the width of the nose of the intended wearer.

In the preferred form, the interface is intended to be of small size and the body portion 303 of the mask curves to follow approximately the contour of the upper lip of the wearer, and the seal is formed such that the opening 403 follows this approximate curve, in plan.

The central portion 407 of die seal extends above and below the opening 403. Above the opening 403, and the nostril locators 401, the central portion includes an outer-facing wall 431, and inward-facing wall 433 and a perimeter portion 435. At least the inwardly-facing wall portion 433 and the outwardly-facing portion 435 are preferably thin and supple.

Below the opening 403, the central portion 407 includes an outer wall portion 441, an inner wall portion 443 and a peripheral portion 445. At least the inner portion 443 and the peripheral portion 445 are preferably thin and supple.

In use, the supple interior wall portions above, below and to each side of the nasal locators are inflated by pressure inside the seal (from the flow of gases supplied to the patient interface) to press against the skin of the wearer and conform to the contours of the outside surfaces of the nose of the wearer and to the surfaces of the lower face of the nose of the wearer and to the surfaces of the upper lip of the wearer immediately below the nose. Movement of the mask body does not break this seal, as the supple perimeter or periphery of the seal allows the mask body to move in the direction of movement to at least a small extent. The supple perimeter de-couples the position of the nostril locators from the position of the mask body, allowing the mask body to displace both laterally and vertically (relative to axes of the patient's face). The side portions 411 engage the sides of the patient's nose, and form some additional seal against them and support the location of the mask.

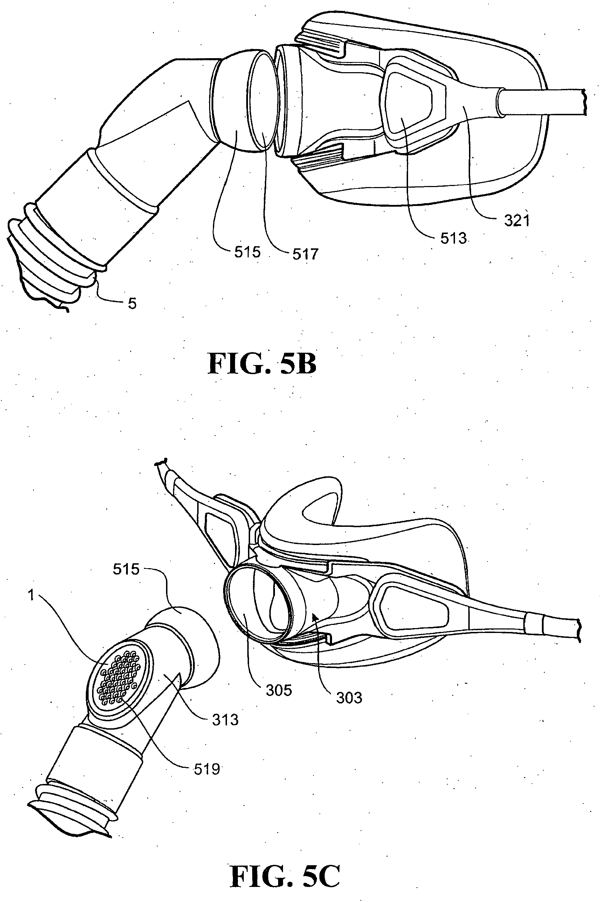

The mask body and seal are illustrated in larger format in Figure 5A and in Figures 5B and 5C. As previously described, the seal includes opening 403, with arrangements to secure the seal to the mask body. The mask body includes a socket 502 for connecting with the supply conduit, and a seal opening 501 for engaging with the opening 403 of the seal. The seal opening 501 and the opening 403 of the seal are provided with complementary features to engage them together. In the illustrated form, the seal opening 403 includes arrangement 507 of lip and channels, and the periphery of the seal opening 501 of the mask body includes a complementary arrangement of 505 of channels and lips. The arrangement of channels and lips is devised to ensure that when the seal is properly fitted to the mask body, and supplied with gases under pressure, there are no leaks at this join. Many other ways can be devised for connecting the seal and the mask body and this arrangement is only illustrative.

In the mask body, the outlet opening 501 to the seal is directly opposite the opening of socket 502, so that the opening 502 is centrally located. Either side of socket 502 extends a central side portion 509. The central side portion 509 may be a plain covering wall, enclosing a portion of the opening 403 of the seal. The central side portions 509 may include small apertures as part of a gas washout vent.

In the preferred embodiment illustrated, side arms 511 extend beyond the extent of the seal. opening 501, best illustrated in Figure 7A, the side arms 511 preferably extend beyond the extreme width of the seal. Each side arm 511 includes a connector portion 513 for connecting to connector portion 321 of the strap. In the illustrated embodiment, the connector portions comprise a securing post with a perimeter undercut. The strap has a small loop formed at each end which stretches over the securing post and engages in the undercut. This connector form is simple and intuitive, but other connector forms may be provided, for example, each side arm 511 could be provided with a portion (for example a male or female part) of a clip.

As best illustrated in Figure 7A, the side arms 511 diverge from the outside wall of the seal, for example at an angle 713 of between 30° and 80°. A strap secured to post 513 leaves the side arm in a manner as illustrated by line 715 in Figure 7A, at a location spaced apart from the seal, and spaced apart from the face of the wearer. This is illustrated by the relative locations of the tip 717 of the side arm 511 compared with the centre line 719 of the interface. The distance 721 from the centre line to the tip of the side arm is preferably between 25mm and 50mm, and most preferably about 45mm. This compares with the distance 723 between the centre line 719 and the central axis of the nasal locator, which is preferably between 5mm and 10mm, and most preferably about 7mm. It also compares with the approximate location of the inner wall surface of the side portion of the seal, where it leaves the central portion of the seal. This location is illustrated by broken line 725 in Figure 7A. Preferably this separation 727 is between 10mm and 20mm, and most preferably about 15mm. For further comparison, the outer most extent of the side portion is illustrated by broken line 729. The displacement 731 of broken line 729 from centre line 719 is preferably between 15mm and 30mm, and most preferably about 25mm.

In the front to back direction, the tips 717 are preferably rearward of the base of the nasal locators, so that bases of the nasal locators are between the central portion of the mask body and a line connecting the tips 717.

With further reference to Figure 7A, overall, the mask seal may have a broadest exterior extent 741 of between 30mm and 60mm and most preferably about 50mm. The seal and mask frame may have an overall depth 743, preferably between 40mm and 65mm and most preferably about 55mm. Within this depth, the interior space defined by the seal, which wraps around the nose of the user in use, may have a depth 745 that is preferably between 20mm and 40mm and most preferably about 30mm.

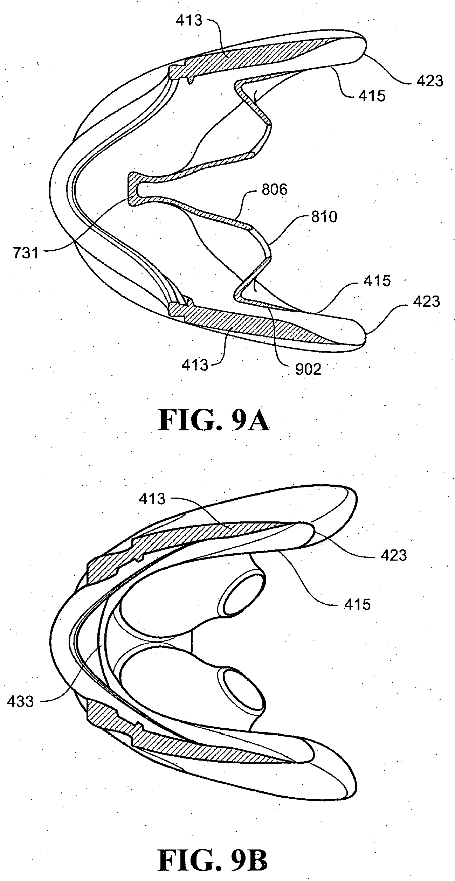

As can be seen from Figure 7A, in overall form, the seal curves through a significant arc such that the side portions are generally parallel with each other and opposed across the space that will accommodate the nose. The orientation planes of the side portions may form together an angle of between 0° and 45° and preferably between 0° and 25°. Preferably this applies to both the inner wall, and the outer wall, as illustrated in Figures 9A and 9B. Preferably this is also true for substantially all of the vertically displaced levels within the seal, as illustrated by the different levels shown in Figures 9A and 9B.

The overall plan form of the seal, as illustrated in Figure 7A, could be considered parabolic, half elliptical, half oval or U-shaped. Viewed generally, the central portion of the seal defines the width of the seal, with the side portions of the seal extending away from the lateral ends of the central portion in a direction substantially parallel with each other and substantially perpendicular to this width dimension.

Figures 5B and 5C also show the connection of the swivel elbow 313 to the mask body 303. The swivel elbow 313 includes a ball portion 515 and opening 517. The outer surface of ball portion 515 is preferably a frustospherical surface, but could be formed with variation and still achieve a substantial seal with socket 502. Similarly, the socket 502 is preferably a frustospherical surface with a slight intruding lip. This is best illustrated in Figure 8, where lip 802 intrudes slightly relative to the remainder frustopherical surface 804.

The swivel elbow 313 preferably defines an angle between flow in the conduit, and flow through the connection to the mask of between 0° and 90°, preferably between 30° and 60°, and most preferably about 45°. The elbow may incorporate apertures 519 forming part or all of a gas washout vent for the patient interface. The apertures are preferably located on the outside of the bend of the elbow, substantially in the line of the flow path of gases leaving the mask.

Figures 5B and 5C also illustrate the connector portions 321 of the strap, engaged over posts 513 of the mask body.

Figures 7A to 7D give context for cross sections illustrated in Figures 8, 7D, 9A and 9B.

Figure 7D is a cross section through line EE of the seal of Figure 7C. Figure 7D illustrates the thicknesses of portions of the seal in the vertical centre plane of the seal. This shows a thickening of the seal in the region 731 immediately adjacent and between the nostril locators. The cross section also illustrates a thickening of the seal in the outer wall portion 431 of the central portion of the seal above the outlet 403 and thickening of the outer wall portion 441 of the central portion below the opening 403. These thickened sections are preferably gradually thickening from the thin supple perimeter portions 435 and 445 respectively to a thickness of approximately 2mm to 4mm. The supple wall portions, being the peripheral portions 435 and 445 and the lower inside wall portion 443 and upper inside wall portion 433 preferably have a wall thickness between 0.05mm and 0.5mm and most preferably between 0.1mm and 0.2mm.

The portion 731 between the nasal locators preferably has a thickness between 2mm and 0.5mm and preferably between 0.8mm and 1mm.

These dimensions are given relevant to a silicone material having a Shore A hardness of about 40. If the seal is formed of other materials commensurate alterations of dimension may be possible while retaining suppleness of the envelope in the regions preferred supple and retaining sufficient stiffness to provide form to the envelope in regions intended to provide shape.

Figure 8 is a section through DD of the mask seal and body of Figure 7A. This illustrates the cross section of the central portion of the seal at the outward side as illustrated and described already in reference to Figure 7D, but illustrates the connection of the seal opening to the opening of the mask body. However, the section of Figure 8 also illustrates a preferred cross-sectional form of a nasal locator. In particular, the thickness of the material of the wall 806 of the nasal locator is preferably between 0.5mm and 2mm and most preferably between 0.8mm and 1mm.

The nasal locator includes a base portion 808 and a nozzle portion 810 with a central opening 812. The portion including opening 812 fits inside the nostril of the user. The base portion 808 provides primary location at the entrance to the nostril.

Figure 9A is a section through line GG of Figure 7D of the seal. This is a cross section of the seal approximately on the horizontal centre plane passing through the nostril locators. This section shows that the wall portions 806 of the nostril locators, the immediately adjacent region 902 outside the perimeter of the nostril locators, and the centre portion 731 between the nostril locators all having a thickened wall relative to the supple wall portions 415 and 423 of the side portions of the seal. In particular, the regions immediately adjacent and including the nasal locators preferably have a thickness between 0.5mm and 2mm and most preferably between 0.8mm and 1mm.

The outer wall portions 413 of the side portions of the seal are substantially thicker again than the portions adjacent the nostril locators. These portions preferably have a thickness between 2mm and 5mm and most preferably between 3mm and 5mm. These portions gradually taper in thickness to reach the supple thickness where they become the peripheral portion 423.

The thickened portion of the outer side wall 413 of the side portions preferably extends to within 10mm of the outer most tip of the side portion.

The thickening and stiffness of these outer side portions 413 provides substantial form to the seal and stability with the seal in place. The side wall resists outward flexing of the side portions of the seal when the seal is inflated under pressure from the supply while the wall portions 413 will flex outwardly under pressure and have sufficient reaction force to retain the seal wrapped around the nose of the wearer.

The stiffening produced by thicker regions of the seal could be provided by a composite material, or combination of parts. For example, the stiffness could be provided by reinforcement in the silicone, or by a flexible insert of stiff material. The insert of stiff material could be integrated to the mask body. Preferably, the construction is such that the side portions of the seal provide resistance to flexing with an effective stiffness of at least 1N force at the end of the stiffened region to flex the side portion through an angle of about 60°.

Figure 9B illustrates a further, substantially horizontal plane, above the cross section of Figure 9A. This cross section is taken through line FF of Figure 7D. This again illustrates the thickness of the outer side wall portions 413 of the side portions of the seal relative to the inner side wall portions 415 and perimeter portions 423 and relative to the thickness of the upper internal wall portion 433.

Again, the side portions 413 preferably have a thickness between 2mm and 5mm, most preferably between 5mm and 3mm. The supple portions 415, 423 and 433 have thickness between 0.05mm and 0.5mm, and most preferably between 0.1mm and 0.2mm.

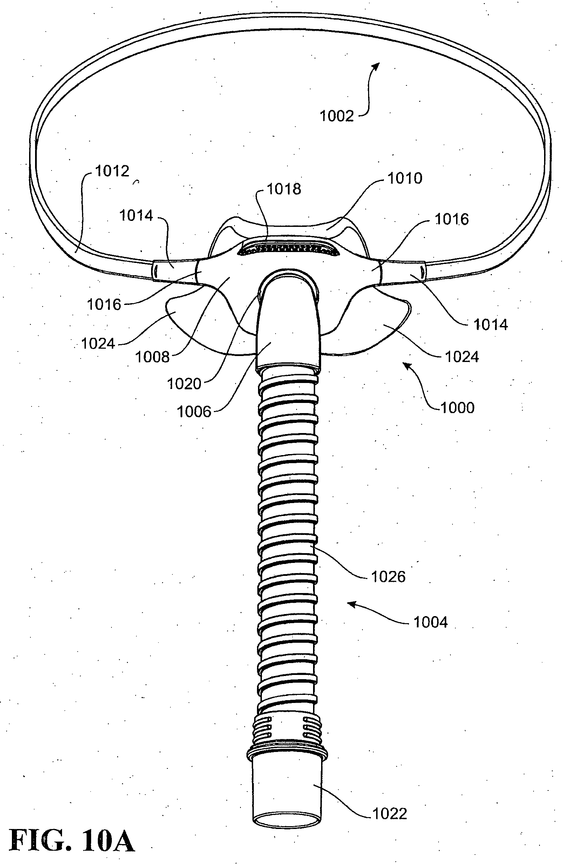

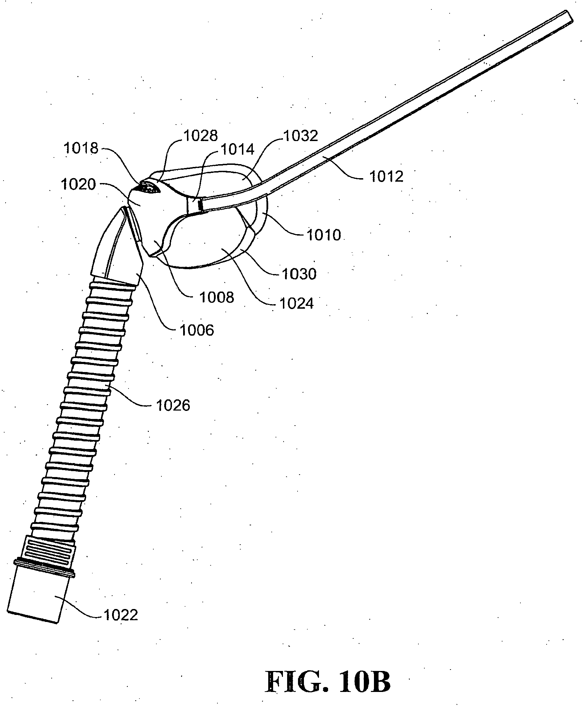

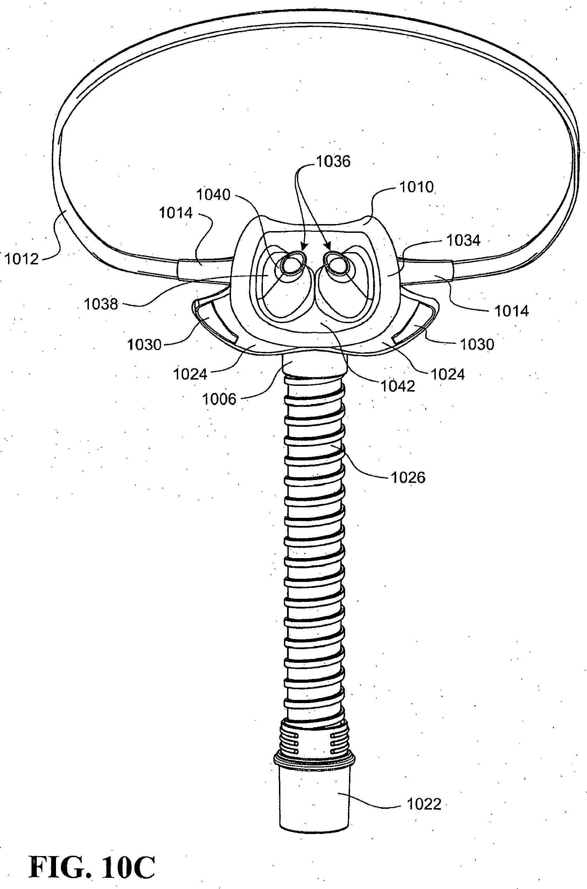

Figures 10A to 10C are perspective views illustrating a patient interface incorporating a number of inventions described in this specification. Figure 10A is a front view, Figure 10B is a profile view, and Figure 10C is a back view.

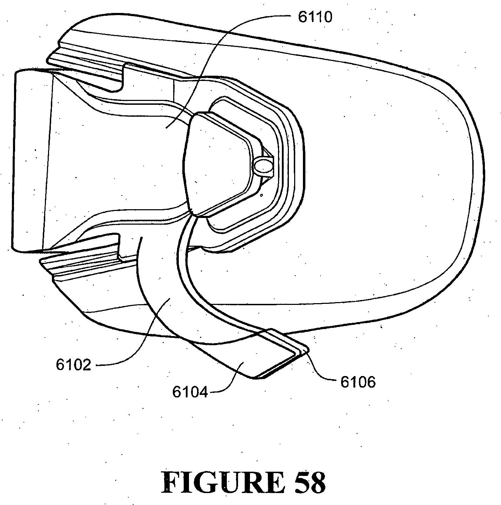

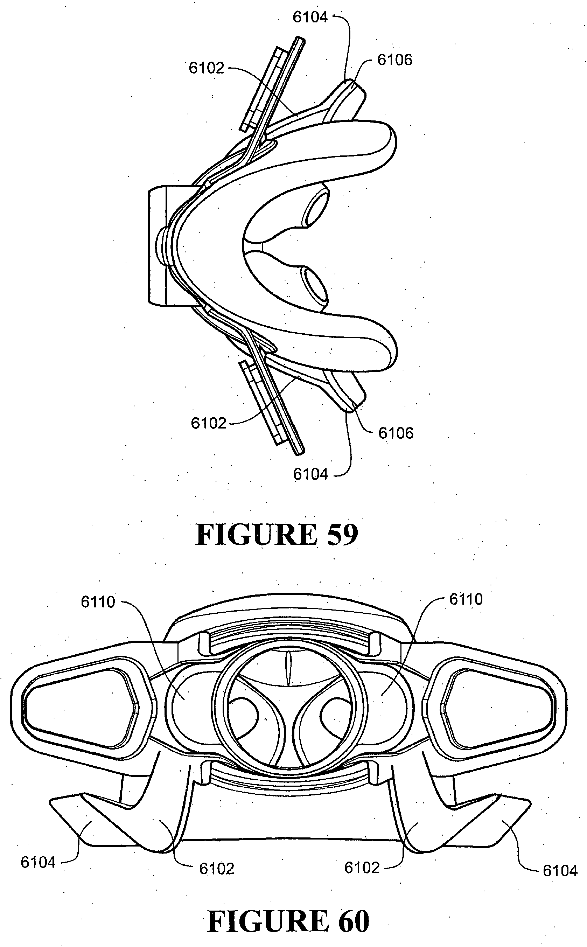

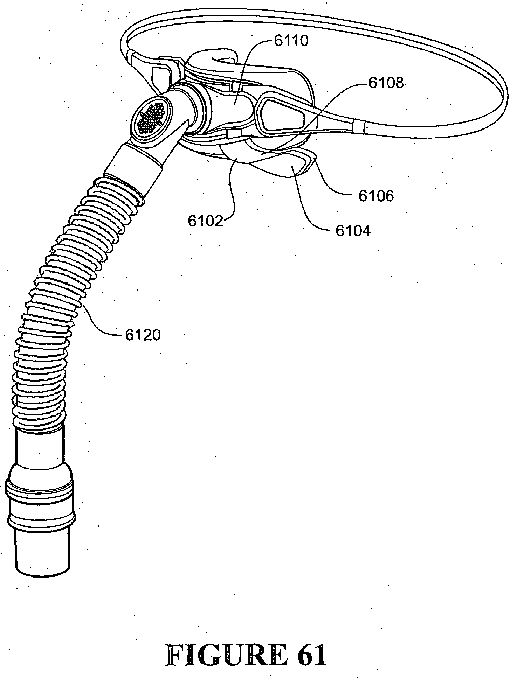

The interface 1000 is similar to the interface described with reference to Figures 1 to 3. The seal 1010 includes many similarities to the seal of Figure 4. The frame assembly 1008 has some of the same features as the frame 303 of Figure 5. Like the interface of Figures 1 to 3 the interface 1000 has a short depending conduit coupled to the frame assembly 1008 by a ball joint or swivel connection. Also like the interface of Figures 1 to 3, the interface is preferably secured to the head of the wearer by a single loop strap. This loop strap may be made of materials described later in this specification. The frame assembly may include lip stabilisers as described with reference to Figures 58 to 61.

Referring to Figures 10A to 10C, the interface 1000 has an overall configuration including a frame assembly 1008 and a seal 1010 on the inside of the frame assembly. A supply conduit 1004 depends from the frame assembly and supplies gases to the frame assembly, and thence to the seal. Headgear 1002 connects to the frame assembly to secure the interface to the head of a user.

The frame assembly includes side portions 1016. Like the assembly of Figure 5 the side portions may extend away from the outside wall of the seal (and therefore away from the face of the wearer when in use). At least one, and preferably both, side portion 1016 provides for connection of a connector 1014 of headgear 1002.

The connector 1014 is connected to terminate an end of the strap 1012. Preferably each end of the strap 1012 includes a connector, and each side portion 1016 includes a matching cooperating connection.

The supply preferably includes a short flexible tube 1026, a supply connector 1022 and an interface connector 1006.

Tube 1026 may take many forms. For example the tube 1026 may be a stretchy conduit, a springy conduit that is usually short in the relaxed condition, an extruded corrugated tube or a wound tape tube with a spiral rib. The tube should be resistant to crushing and allow extensive lateral turning and bending. The tube is preferably between 50mm and 150mm long.

The interface connector may include a bias flow vent. However in the illustrated interface no bias flow vent is provided on the connector. The connector 1006 preferably includes portions of a swivelling joint 1020 to the frame assembly 1008. The connector preferably includes a portion of a ball joint or swivel joint, and most preferably includes an extending hollow plug portion which extends into a socket of the frame assembly.

With reference to Figure 10B the connector preferably turns the supply flow through an angle between 25 degrees and 75 degrees. Most preferably the connector turns the flow through an angle between 40 degrees and 60 degrees. This angle combines with the angle of the front face of the frame assembly in the immediate periphery of the joint 1020 (for example relative to the coronal plane of the wearer) so that the tube 1026 hangs easily below the interface (when the user is upright), remains close to the user when the user is lying down, keeps the joint 1020 close to the patient.

The supply connector 1022 preferably includes a swivel.

The frame assembly 1008 preferably includes a bias flow vent 1018. Bias flow vent 1018 may include at least flow path from the interior of the interface to ambient surroundings. An interface may be provided for use with a return flow type ventilator not including any bias flow vent.

Where a bias flow vent is included, the bias flow vent may include at least one aperture extending through the wall defining the frame assembly. In the illustrated interface the bias flow vent 1018 includes a plurality of apertures. The apertures are arranged to direct the outlet flow of gases predominantly upward relative to the head of the wearer. By predominantly upward we mean that the gases are directed in a direction that is more parallel to the plane of the face (or the coronal plane) than perpendicular to the plane of the face, and generally in the direction toward the forehead.

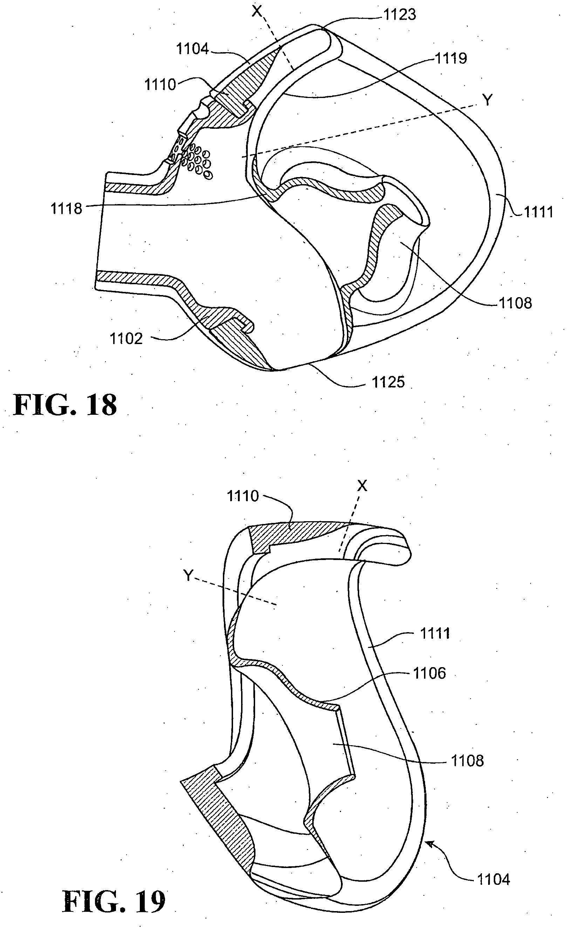

As best seen in Figure 10C, the frame assembly may include depending support portions 1024 which extend below the seal 1010. The depending support portions include pads 1030 which are intended to provide support onto the upper lip region of the wearer, in the vicinity of the nasolabial folds. On some users, the pads 1030 may extend onto the lower cheek region immediately adjacent the nasolabial folds. The pads are intended to contact the face only when required to support the seal. This aspect of the interface is covered in more detail with reference to Figures 18 to 61.

Also in Figure 10C, the seal can be seen to include a pair of nasal locators 1036. Each nasal locator includes an opening, which in use is disposed within a nostril of the wearer. The nasal locators extend from a supple inner wall portion 1042 of the seal.

In the illustrated interface each nasal locator includes a generally tubular nozzle portion 1040 and a convex base portion 1038. The generally tubular nozzle portion 1040 may be somewhat tapered toward the open end. The convex base portion may be considered a dome shape.

In some embodiments, the convex base portion extends all of the way around the locator and surrounds the tubular nozzle portion. In other embodiments, the convex base portion may extend most of the way around, but not completely around the locator. For example, the base portion may extend continuously 60% or more of the way around the locator.

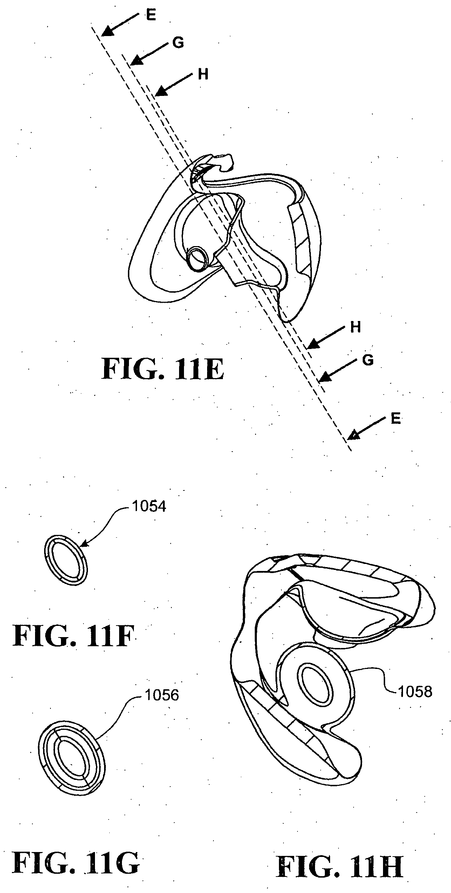

Both the nozzle portion and the base portions of each locator are preferably oval or elliptical in cross-section. This is illustrated in more detail in Figures 11E to 11H.

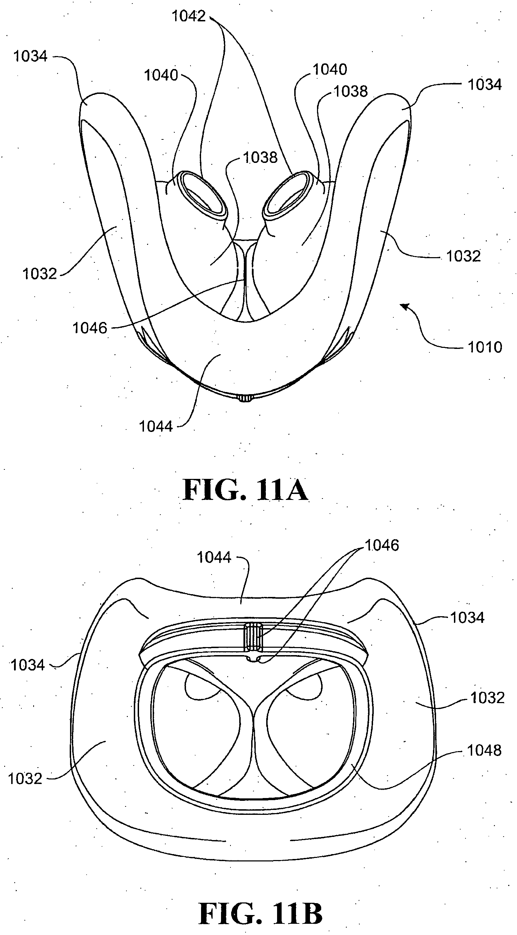

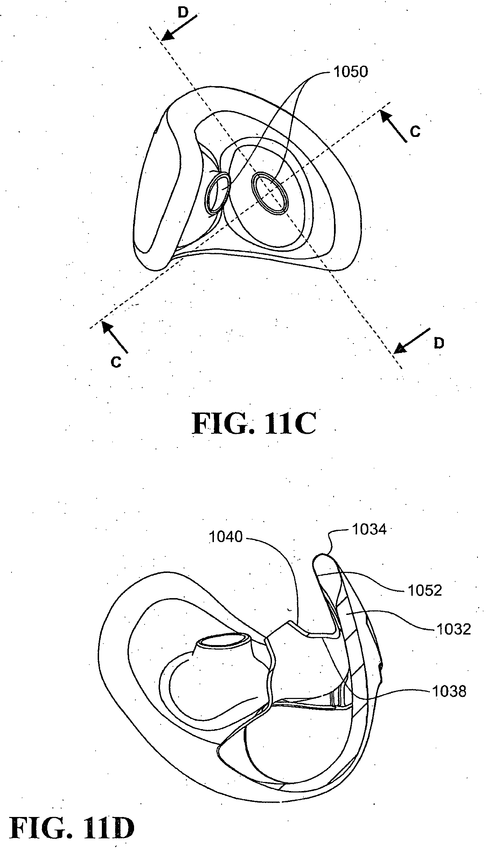

Figures 11A to 11H are views of the soft seal component of the interface of Figures 10A to 10C. Figure 11A is a top view. Figure 11B is a front view. Figure 11C is a rear view taken from a position directly facing the open end of a nasal locator. Figure 11D is a cross section through section CC in Figure 11C.

From these figures many similarities can be seen to the seal of Figure 4. The utility and function of the similar features will not be repeated in detail.

In general terms the seal includes in inward facing wall portion and an outward facing wall portion. The inner facing wall portion forms the seal against the face surfaces of the wearer. The outer facing wall portion connects the seal to the frame assembly and supports the overall shape of the seal. Like the seal of Figure 4, the general shape of the seal is a U-shape or V-shape, with side portions extending rearward from a central transverse portion.

The outward facing portion includes side portions 1032, which are of greater cross section than the inward facing wall. The inward facing wall includes nasal locators supported on a generally supple background 1052. A supple periphery 1034, 1044 connects the outward facing wall to the inward facing wall around the perimeter.

With particular reference to Figure 11A, and to Figure 11D, each nasal locator preferably comprises a tubular portion 1042 and a dome portion 1038.Furthermore each tubular portion terminates in a rim 1042 defining opening 1050.

The tubular portion tapers toward the opening 1050. The taper is preferably a linear taper. The taper may alternatively be a curved taper. The surfaces of the dome portion and the tubular portion meet at an included, obtuse angle, for example between 120 degrees and 160 degrees.

The cross-section of Figure 11D suggests that the tubular portion 1042 is approximately frusto conical, and the dome portion 1038 is approximately frusto spherical. However in each case the alternate cross section (Figures 11F to 11H) is oval rather than circular. Accordingly the form of the dome may be regarded as ellipsoid. The form of the tubular portion may be regarded as an elliptic cone. However in each case the shape need not be a true ellipse, and may instead comprise less regular oval shapes, such that the form of the dome might be regarded an ovaloid or ovaloid dome, and the form of the tube an ovoid or ovaloid cone or a tapering ovoid or ovaloid tube.

The tubular portions of the nasal locators are spaced apart, but aligned such that their axes are generally convergent. Preferably the convergent distance is between 10mm and 40mm from the openings of the nasal locators.

The dome portions of the nasal locators come close together, about or adjoin at a cleft 1046 at the centreline of the seal. Preferably the cleft 1046 is formed to allow some freedom of movement of the dome of one nasal locator relative to the dome of the other nasal locator.

With particular reference to Figures 11E to 11H, the oval cross section of each nasal locator is shown in more detail. In particular three cross sections of the nasal locator are shown. These cross-sections are taken on planes parallel to the plane of the opening of the open end of the nasal locator.

What can be seen from these cross sections is the oval form. In particular the oval or ellipse of the opening 1050 has an aspect ratio of approximately 1.8 to 1. The major axis of the oval or ellipse is greater than twice the minor axis, and preferably between 1.5 and 3 times the minor axis.

The oval form is retained throughout the tubular portion and the dome portions of the nasal locator. However, in the illustrated seal the absolute difference in the major and minor axes is retained, rather than the ratio. Therefore the ratio reduces moving from the shape of opening 1050, to the first cross section 1054 spaced toward the base of the tubular portion. The ratio further reduces moving from cross section 1054 to cross section 1056 at an intermediate position on the dome portion. The ratio further reduces moving from cross section 1056 to cross section 1058 at the base of the dome portion.

By way of example, for the tip opening the major axis dimension may be about 9mm and the minor axis about 5mm. For the cross-section of the tubular portion at EE, the major axis dimension may be about 13mm and the minor axis dimension about 9mm.

For the dome portion at GG, the major axis dimension may be about 18mm and the minor axis dimension about 15mm.

For the dome portion at HH, the major axis dimension may be about 22mm and the minor axis dimension about 18mm.

As is best illustrated in Figure 10C, the oval forms of the nasal locators are preferably not aligned parallel when viewed from the back of the interface. In particular the upper ends of the ovals are closer than the lower ends. When projected posteriorly onto a coronal plane of a hypothetical wearer the major axes of the ovals form an angle of between 60 and 120 degrees.

Each nasal locator is preferably provided with a lip 1042 at the open end 1050. The material at the lip 1042 is preferably thicker than the material of the rest of the tubular portion, or of the material of the region of the tubular portion in the near vicinity of the lip. The thickened lip provides some positive form to the end of the locator when locating the locator in the nostril of the wearer. This may be useful for the wearer to better feel the end of the locator when fitting the interface.

Figures 11B and 11E also illustrate features of the seal involved in securing the seal to the frame assembly. As seen in Figure 11B, the outward facing portion of the seal includes a major opening for securing with the frame assembly. The major opening includes a lip 1048 that runs the perimeter of the opening. The lip may be constituted by the thicker regions of the outward wall of the seal. For example the lower portion of the lip is illustrated in cross section in Figure 11E.

At a location, or multiple locations, on the lip, markers or features may be provided to ensure the correct assembly of the seal relative to the frame assembly. The seal illustrated and described is sensitive to incorrect assembly. In this embodiment the seal is arranged to be detachable from the frame.

For easier positioning, the lip is preferably provided with a protrusion, which engages in a notch of the frame when the seal is in the correct position. The lip may be provided with a protrusion 1046 extending in either or both of the inward and outward direction. In the illustrated embodiment two protrusions 1046 are provided, which engage with notches 1074 and 1092 of the frame.

The lip of the seal takes the overall form of a bent oval. By this we mean an oval that has been bent so that the ends of the oval are curved away from a plane tangential to the central portions of the oval. This can be seen in a general sense in the form of the outward wall portion of the seal, in Figure 11A.

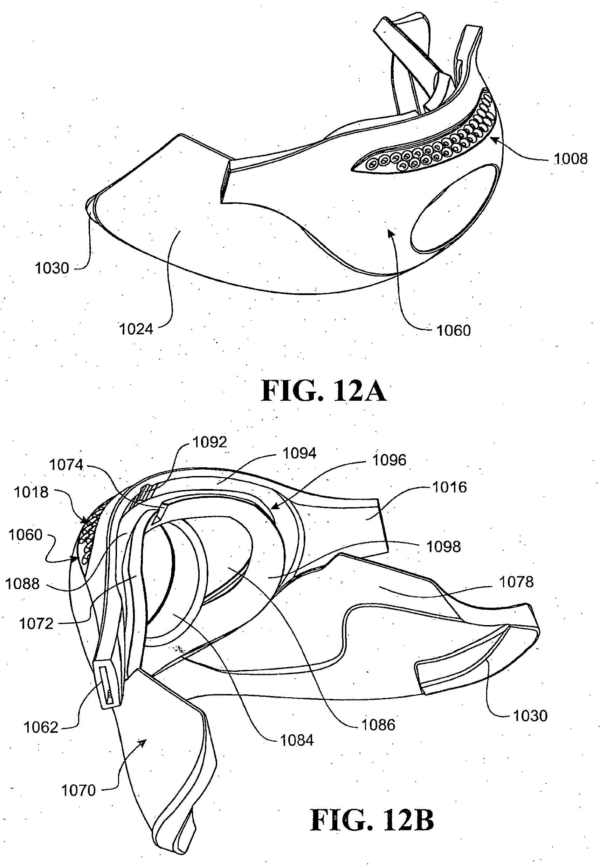

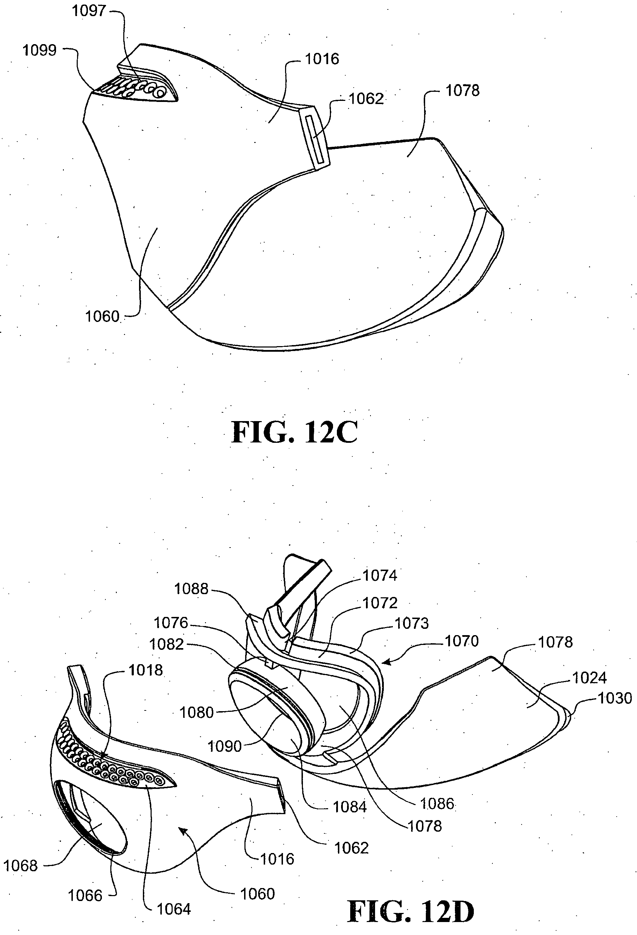

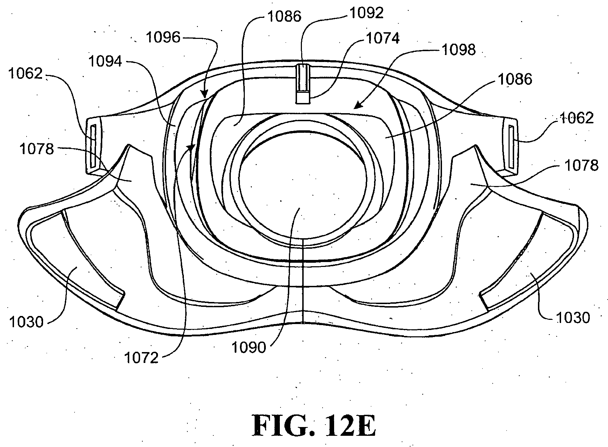

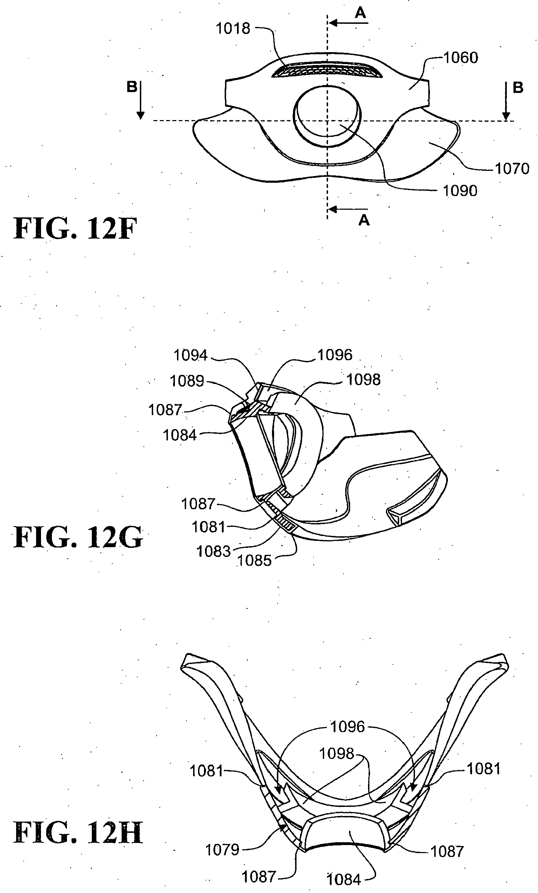

Figures 12A to 12H are views of a frame assembly of the interface of Figures 10A to 10C. Figure 12A is a front perspective view of the frame assembly. Figure 12B is a rear perspective view of the frame assembly. Figure 12C is a side profile of the frame assembly. Figure 12D is an assembly view of two components of the frame assembly. Figure 12E is a back view of the frame assembly.

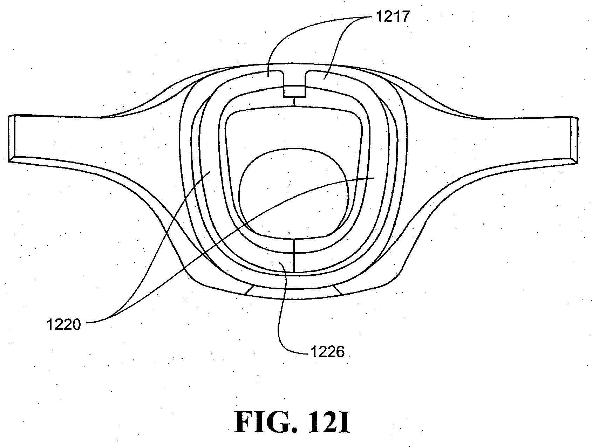

Figure 12I is a back view of a frame assembly that is of a slightly different configuration, and with the stabiliser portions not showing. This illustrates an alternative configuration of the channel for securing the seal. The frame assembly may otherwise be formed and assembled in substantially the same manner as the frame assembly of Figures 12A to 12H.

The frame assembly 1008 comprises two major components, a first component 1070 and a cover 1060.

The two components of the frame assembly combine to provide a channel 1096 for securing the lip 1048 of the seal.

The first component 1070 may include a first portion of the channel, while the cover 1060 provides a second portion of the channel. The component 1070 may provide an inside flange portion 1072, and the cover provide an outside flange portion 1094. A base portion for the channel may be provided extending from the flange portion of either part. For example the base portion 1088 may be provided from the flange portion 1072.

The flange portion 1072 may include a notch 1074 for receiving a locating feature of the lip 1048 of the seal. The flange portion 1094 may include a notch 1092 for receiving an additional locating feature of the lip of the seal.

According to the preferred form of the seal and channel the channel has a general outward flare, so that portions of the channel on opposite sides of the main opening diverge relative to each other when progressing from the base of the channel to the open edge of the channel. Accordingly the lip of the seal stretches to pass over the inner flange portion 1072 and the seal is retained in the channel by the elastic tension of the lip.

In the frame assembly of Figures 12A to 12H, the channel forms a general bent ellipse. In the example of Figure 12I, the channel follows a path more alike to a bent rounded oblong or trapezoid. In particular, the overall path of the channel is not as broad as the path of the channel in the embodiment of Figures 12A to 12H. Furthermore, the channel includes comparatively straight side portions 1220. The side portions 1220 converge slightly extending toward the lower portion 1226 and diverge slightly extending toward the upper portion 1217. While this assembly retains the overall bent oval shape, the straightened side portions are found to improve ease of assembly in comparison with the sharply curved end portions of the bent ellipse of Figures 12A to 12H.

The notch and protrusion alignment is preferably provided adjacent the upper or lower portion of the channel, most preferably adjacent the upper portion of the channel. Thus the lip 1048 can be seated in the channel with the correct location, and the rest of the lip then stretched over the inner flange portion 1072.

The first component 1070 may include extensions to carry support pads 1030. For example a lower shield portion may extend in a curve below the supply opening of the interface. The lower shield portion may include side portions. Pads 1030 are provided on the lower inside surface of the side portions.

An internal surface 1078 of an upper portion of the shield may wrap around to the sides of the interface and be located outside and adjacent the outward wall of the seal. The upper portions of the shield can assist in retaining the form of the seal under internal gases pressures, by constraining outward deflection of the outside of the seal envelope.

The first component may carry a portion of the swivel connection 1020. Preferably the first component 1070 includes a ring portion 1080, open to the front and the back. The ring portion preferably includes surfaces to engage with surfaces of the supply tube connector.

Preferably the ring portion 1080 includes an inwardly facing substantially spherical surface 1084. The inwardly facing surface 1084 may engage an outwardly facing complimentary surface of a ball portion of the supply tube connector. The ball portion of the supply tube connector may have in internal passage leading gases from the supply tube through the ring portion 1080.

One advantage of this arrangement is improved available material choices. The supply tube connector is preferably made from a different base polymer material to the ring portion 1080 of the component 1070. Different polymer materials working against one another tend to exhibit lower friction and noise than polymers of the same base material. Yet the outside cover 1060 can be formed from the same material as the supply tube connector, and thus present an aesthetically pleasing exterior.

For example the ring portion of component 1070 may be formed from a material chosen from its strength and toughness, such as a polycarbonate plastic, and the cover 1060 and supply tube connector formed from a plastic chosen primarily for surface finished and appearance such as an acetal plastic. The acetal plastic may include a lubricating copolymer such as PTFE (polytetrafluroethylene).

The ring portion 1080 may also provide features for securing the two components 1060 and 1070.

For example the outer surface of ring portion 1080 may be provided with outwardly facing features such as depressions, or a channel 1082, to cooperate with inwardly facing clips or an inwardly facing annular ridge 1087 of the perimeter 1066 of opening 1068 of cover 1060. This is best illustrated in the cross section in Figure 12G.

The outer periphery of the cover 1060 may blend into the surfaces of the component 1070 in some parts. For example the lower curved edge of the cover 1060 may abut the upper curved edge of the lower shield of the component 1070. This is indicated at 1081 in Figure 12G. A similar abutting edge at the side portions is indicated at 1081 in Figure 12H.

The components 1060 and 1070 may form an outlet plenum adjacent the bias flow vent 1018. This is best understood by considering the exploded view Figure 12D and the rear view of Figure 12B ad the cross sections of Figures 12G and 12H. The plenum 1079 is defined between a bent oval frame portion 1072, of component 1070, and the inside surface of the cover 1060 in the vicinity of aperture 1068.

Openings 1086 into the plenum 1079 are defined by the rear edge of the ring portion 1080 and by the oval frame portion 1073. Gases flowing from the seal to the bias flow vent pass through these openings. The inside surface 1098 of the flange 1072 of oval frame 1073 preferably converges moving toward the plenum, defining a flow directing surface acting like a funnel.

The holes of the bias flow vent 1018 lead from the plenum 1079.

The ring portion 1080 opens essentially centrally through the oval frame portion 1073. This divides the flow into the seal and the flow out of the seal essentially at a location directly adjacent the open end of the seal.

The ring portion may be supported in this position by one or more supporting struts 1076, or by regions at the top and bottom where the ring and oval frame portion come together.

The bias flow holes 1097 are arranged in a curve around the upper portion 1089 of the plenum chamber 1079. This upper portion is quite confined in the location illustrated in Figure 12G, immediately above the ring portion 1080, but more open to either side.

To provide an upward exit flow (generally parallel to the coronal plane of the wearer), the bias flow holes may be arranged in a shelf portion 1099 of the wall of the cover 1060.

The lower shield portion of the component 1060 may include a soft covering 1085, such as an overmoulded layer of a soft biocompatible material, over a more rigid skeleton or frame 1083. A preferred covering would be, for example, a thermoplastic elastomer, a polyurethane foam, or a silicone elastomer. The skeleton or frame 1083 of component 1060 may be formed from a copolymer including an amount of a plastic material that bonds strongly with the covering material. For example, the frame 1083 may be formed from a polycarbonate siloxane copolymer. The siloxane contributes chemical resistance and improves the bonding of the overmoulded silicone cover.

Extreme side portions 1016 of the cover 1060 diverge from the outer surfaces of the shield of component 1070. The side portions 1016 end in sockets 1062 for receiving connectors of the headgear strap.

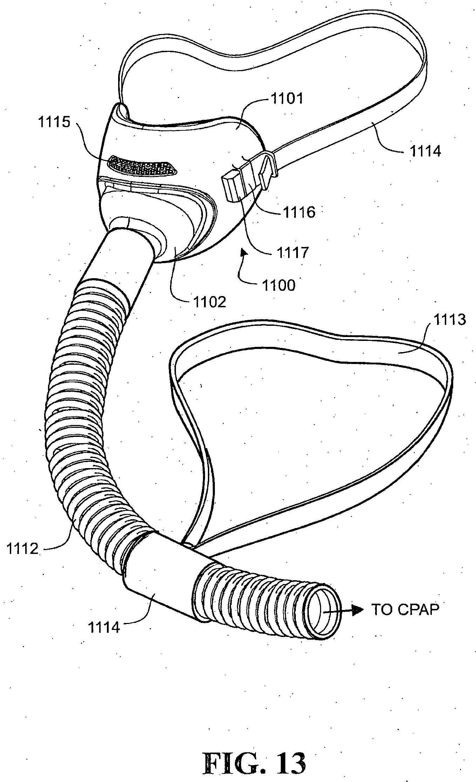

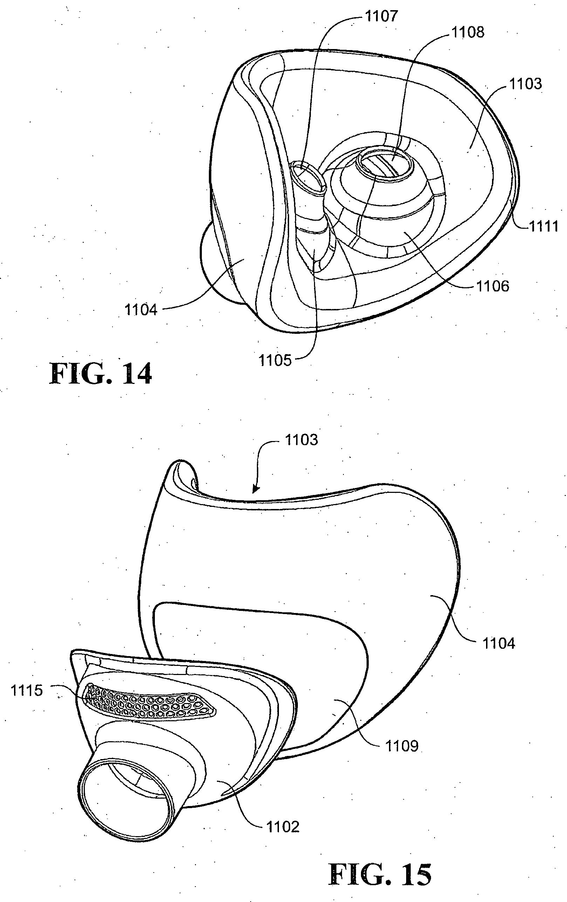

Figures 13 to 20 show a further embodiment of an interface with a wrap around inflating seal. The interface 1100 includes a seal body 1101 and frame 1102.

Attached to the frame 1102 is tubing 1112 that is attached to a gases supply apparatus. The tubing supplies gases to the mask frame and seal. The tubing 1112 may be tethered to the user (wearing the interface 1100) by a lanyard 1113. In use the lanyard 1113 extends about the user's neck. The lanyard 1113 is affixed to the tube by known methods, however, shown is a c-shaped clip 1114 attached to the lanyard 1113 that clips about the tubing.

The interface 1100 is held in place over the user's nose by way of a head strap 1114. The strap is preferably made of a flexible type material, such as silicone or a laminated material well known in the art of head gear straps. Each end of the strap 1114 is preferably fitted to a clip 1116 that attaches to a corresponding eyelet 1117 formed in or attached to the seal body 1101. The strap may be a flat moulded silicone strap, a small hollow silicone tube, or appropriate configurations as are known in the art.

The frame 1102 may have formed in it a plurality of bias flow holes 1115 to allow for exhausting of the user's exhalated gases from the interface. Alternatively, bias flow holes may be formed in the seal body 1101 to allow for exhausting of gases.

The seal body 1101 is again a supple or inflatable type seal. The seal body 1101 is curved in shape to match the contours of a human face and extends about the user's nose, wrapping the user's nose. The seal body 1101 preferably extends completely over the side of the user's nose and may also extend partially over the user's cheeks. The seal 1101 comprises an inner wall with an inner 1103 and an exterior wall with an outer surface 1104. Projecting from the inner surface 1103 are nasal locators 1105, 1106, each with outlets 1107, 1108.

As with the embodiment of Figures 10 to 12 the seal body 1101 includes an inlet opening 1109 that is opposite the nasal locators 1105, 1106 and receives the frame 1102.

The seal 1101 has a variable wall thickness such that there is rigidity around the portions of the seal that project out, and flexibility between the nasal locators 1105, 1106 and a periphery 1110 of the inlet opening 1109 of the seal. This means there is a decoupling effect between the nasal locators 1105, 1106 and the inlet periphery 1110, and subsequently to the mask frame 1102. This will mean that some movement of the mask frame will be possible without disrupting the sealing of the nasal locators 1105, 1106 on or in the user's nostrils.

As can be seen in Figure 18 the inlet periphery 1110, which defines the gases inlet 1105 to the seal, has a substantially thick cross-section. This provides rigidity to the inlet periphery 1110. Similarly, the nasal locators 1105, 1106 have a substantially thick cross-section. However, the thickness of the nasal locators may not necessarily be as thick as or thicker than the inlet periphery 1110. In the preferred form the thickness of the nasal locators is less than that of the inlet periphery.

The areas between the inlet periphery 1110 and the nasal locators 1105, 1106 is preferably thinner in cross-section than both nasal locations and the inlet periphery. For example, in Figure 18 the length of the seal 1101 between X and Y is formed to be substantially thinner in cross-section than either the inlet periphery or the nasal locators. This means this length is more flexible, effectively allowing more movement of the nasal locators 1105, 1106. Furthermore, as the length between X and Y, including an outer periphery 1111, is thinner in cross section the seal will easily inflate to assist in the sealing of the seal about the user's nose.

As shown in Figure 18, preferably a region 1118 of the envelope adjacent to the base or root of each nasal locator has a thickened cross section. As shown, preferable the length or region 1119 of the seal between region 1118 and the thickened inlet periphery 1110 is formed to be substantially thinner in cross-section than either the inlet periphery 1110, the nasal locators 1105, 1106 or region 1118.

In an alternative embodiment, the envelope includes a thickened region 1118 adjacent the root of the nasal locators and the thickness of the cross section of the nasal locators is not thickened. For example, the thickness of the cross section of the nasal locators may be similar to the thickness of the envelope region 1119 extending between the region 1118 adjacent the nasal locators and the inlet periphery 1110. The thickened region 1118 adjacent the root of the nasal locators prevents the base of the nasal locators from deforming or ballooning excessively under typical CPAP pressure. However, the thinner wall of the nasal locators 1105, 1106 in this embodiment may balloon under CPAP pressure.

Preferably the seal 1101 is formed from silicone with a Shore A hardness of about 40. Alternatively other materials with similar properties may be used. For silicone with a Shore A hardness of 40, or other material with similar properties, the thickness of the envelop region 1119 extending between the nasal locators and the inlet periphery is less than approximately 0.5mm. Preferably this region 1119 has a thickness of 0.1mm to 0.2mm. Alternatively this region 1119 of the envelope may have a thickness of less than 0.1mm, for example 0.05mm.

The thickness of the region 1118 adjacent the base of the nasal locators preferably has a thickness of less than 2mm. Preferably the thickness of the region 1118 adjacent the base of the nasal locators is approximately 0.8mm to 1.0mm. Alternatively the thickness of the region 1118 adjacent the base of the nasal locators may be less than 0.8mm, for example 0.5mm.

The thickness of the region adjacent the inlet periphery is approximately 3mm to 5mm, but could be thinner, for example 2mm.

The nasal locators have a thickness of less than 2mm. In the preferred embodiment, the nasal locators have a thickness of approximately 0.8mm to 1.0mm. Alternatively the thickness of the nasal locators may be less than 0.8mm, for example 0.5mm.

In the alternative embodiment described above, the thickness of the nasal locators is similar to the thickness of the envelope region 1119 extending between the nasal locators and the inlet periphery. In this embodiment, the nasal locators have a preferred thickness of approximately 0.1mm to 0.2mm. Alternatively the thickness of the nasal locators may be less than 0.2mm, for example 0.05mm.

Preferably the change in thickness from one region of the seal to another occurs gradually. For example, the thickness of the seal changes gradually from thickened portion 1118 to thinner portion 1119. Similarly, the thickness of the seal changes gradually from thickened portion 1110 to thinner portion 1119.

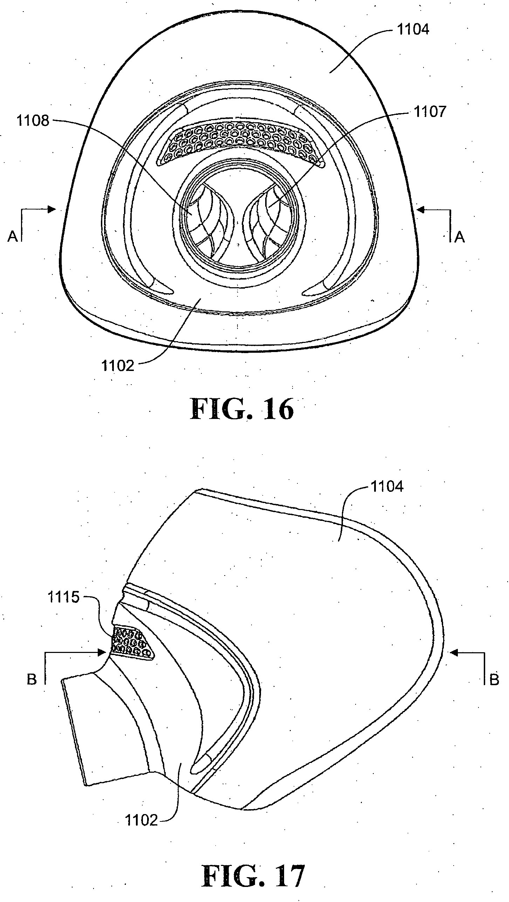

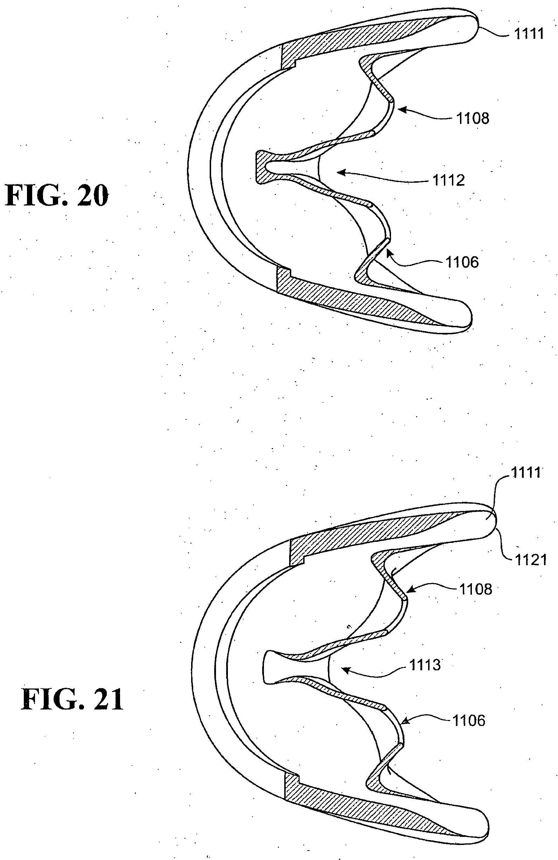

Referring now to Figure 20, which shows the seal 1101 in cross-section through AA of Figure 16. This figure shows an alternative view of the seal 1101 showing the varying thicknesses of parts of the seal. In particular, the inlet periphery 1110 and nasal locators 1106, 1108 are thick in cross-section compared to the outer periphery 1111. At least in the lateral direction, the thickened region 1110 adjacent the inlet extends for at least half of the distance from the inlet to the outer peripheral edge 1121. In the upward direction the thickened region extends at least half the distance from the inlet to the top peripheral edge 1123. In the downward direction the thickened region extends at least half the distance to a lower face portion 1125. The areas of the seal between the nasal locators 1106, 1107, generally indicated as 1112, are also thicker in cross-section to provide additional stability in these areas for the nasal locators.

In Figure 21 an alternative embodiment of the seal is shown. In this alternative embodiment the areas between the nasal locators, indicated as 1113, are substantially thinner in cross-section than that of the inlet periphery 1110 and nasal locators 1106, 1108. This configuration would provide additional flexibility between the nasal locators 1106, 1108.

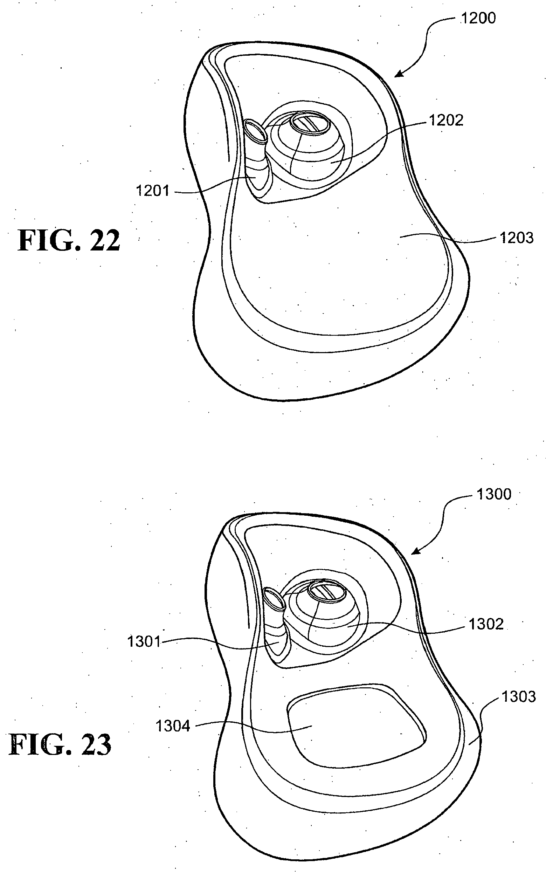

A yet further embodiment of a seal of the present invention is shown in Figure 22. Here the seal is an inflatable type, but the seal extends down to occlude the user's mouth in use. This seal 1200 has nasal locators 1201, 1202 and is received in a frame similar to that described in any of the embodiments detailed above. The seal 1200 has an extension 1203 that goes over the user's mouth creating a seal and reducing mouth leaks.

Another embodiment of a seal of the present invention is shown in Figure 23. This seal 1300 is of the same form as that in Figure 22, with nasal locators 1301, 1302 and a mouth covering extension 1203, but includes an outlet 1304 directed to the user's mouth, allowing for gases to be delivered simultaneously to the user's mouth as well as the user's nasal passages through the nasal locators 1301, 1302.

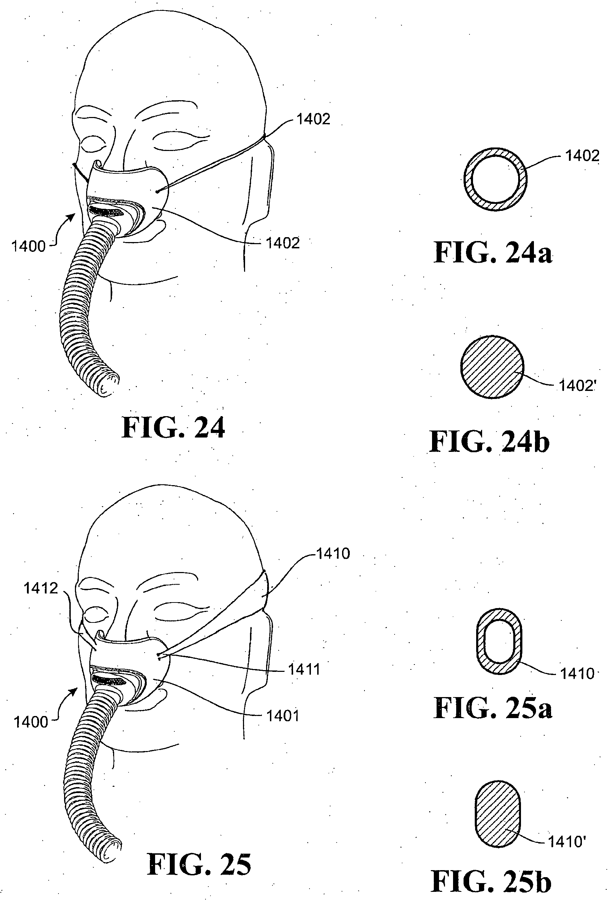

Figures 24 to 29 show various head straps that might be used with any of the embodiments of the interfaces described herein.

Figure 24 shows a single head strap 1402 attached to the interface 1400, particularly to the flexible and inflatable seal 1401 by any appropriate means as known in the art of interfaces and head straps. The strap 1402 may be a hollow tube 1402 as shown in Figure 24a or a solid tube 1402' as shown in Figure 24b. The hollow tube could, for example, be an extended silicone tube, with a diameter between 3mm and 6mm and a wall thickness of 0.2mm to 1mm.

Figure 25 shows a single head strap 1410 attached to the interface 1400, particularly to the flexible and inflatable seal 1401 by any appropriate means as known in the art of interfaces and head straps. The strap 1410 may be a hollow elongated tube 1410 as shown in Figure 25a or a solid elongated tube 1410' as shown in Figure 25b. The strap is preferably thinner in width at its ends 1411, 1412 that attach to the seal 1401 and thicker in width at its midpoint that sits at the back of the user's head in use.

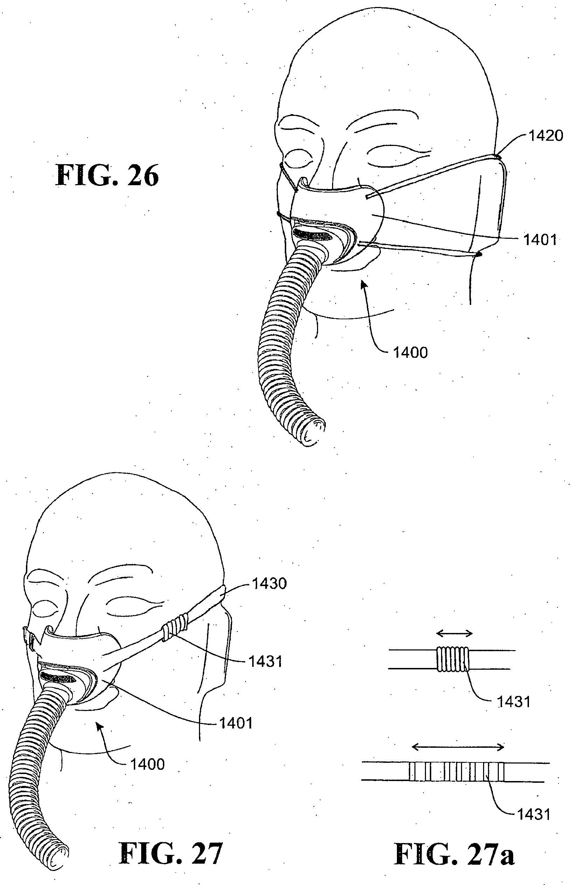

Figure 26 shows a double head strap 1420 attached to the interface 1400. The strap 1420 extends about the user's ears and has two attachment points on each side of the seal 1401 where the strap attaches to the seal 1401.

Figures 27 and 27a show an extendible head strap 1430 attached to the interface 1400. The strap 1430 has an area 1431 that can be extended and contracted to better fit the strap to the user's head.

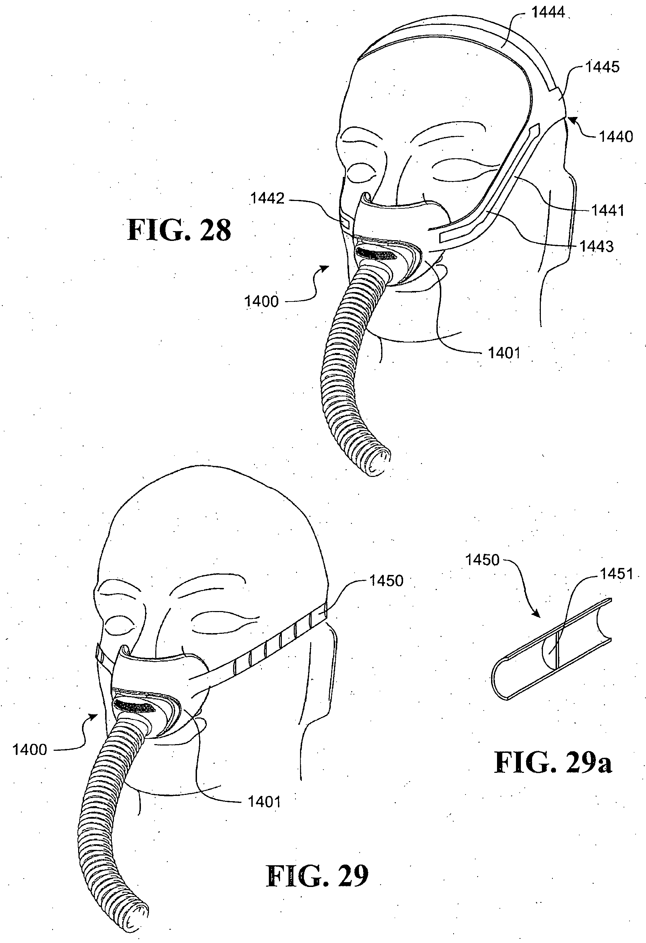

Figure 28 shows an alternative head strap 1440 attached to the interface 1400, particularly to the seal 1401. The head strap 1440 preferably includes side straps 1441 having areas of rigidity 1442, 1443, to provide additional stability to the side straps 1441. The head strap 1440 also preferably includes a top strap 1444 and a back strap 1445 that each extends over the head or behind the head respectively. This head strap is detailed further in US Patent Application No. 12/307993 of Fisher and Paykel Healthcare, the contents of which are incorporated herein by reference.

Figures 29 and 29a shows yet a further alternative head strap 1450 attached to the interface 1400, particularly to the seal 1401. The head strap 1450 is curved and has partitions 1451 that provide additional support or rigidity to the strap.

The head straps detailed above may be formed of any appropriate material, such as, flexible plastics, silicone, laminated fabrics, or other appropriate materials.

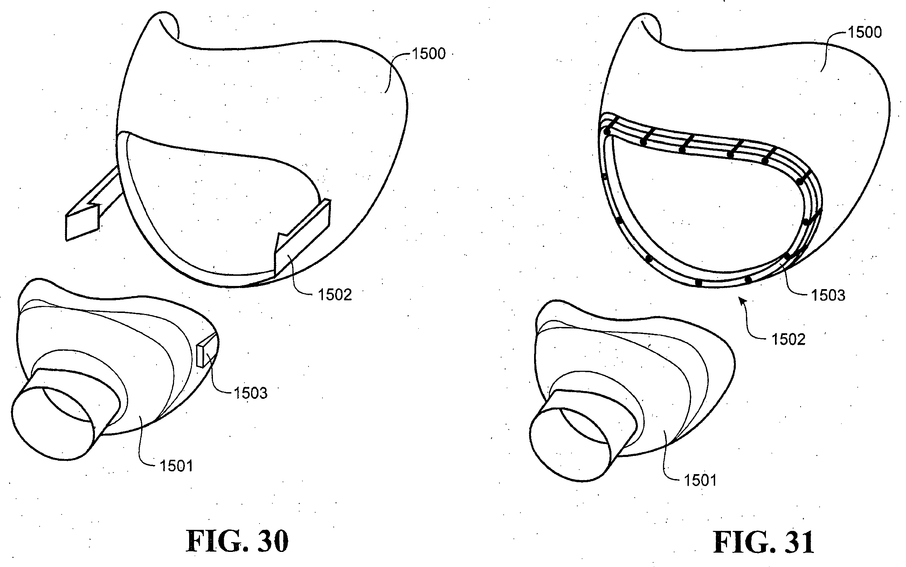

Figures 30 to 33 show various ways in which an interface frame may be attached to an inflatable seal body. In Figure 30 the seal body 1500 includes overmoulded or bonded rigid plastic barbs 1502. The barbs 1502 clip into correspondingly shaped recesses 1503 formed in the frame 1501 and hold the seal body 1500 in sealing engagement with the frame 1501.

Similarly, in Figure 31 the seal body 1500 has a periphery 1512 that is formed with a overmoulded or bonded rigid plastic looped clip 1513 that clips to the frame 1501. Further details of such a clipping mechanism are described in US Patent Application Number 12/502528 of Fisher and Paykel Healthcare, the contents of which are incorporated herein by reference.

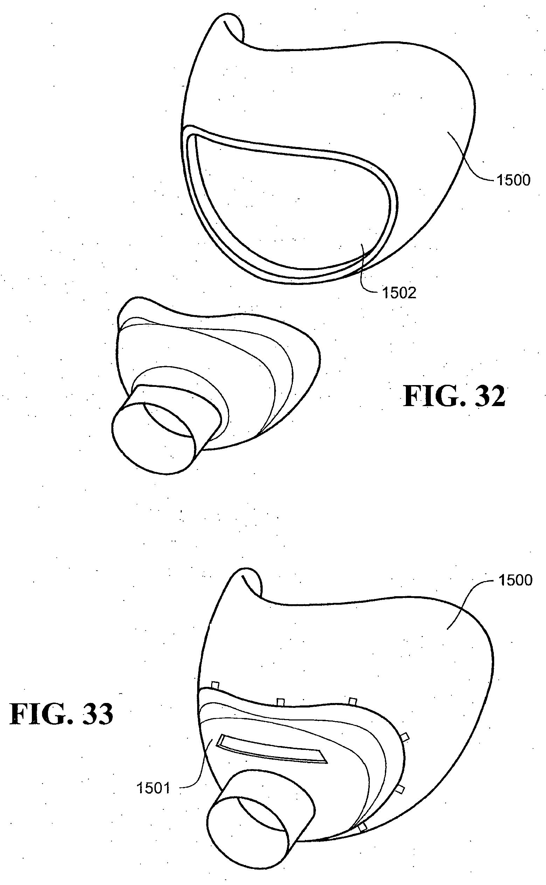

Alternatively, as shown in Figure 32 the seal body 1500 may have an inlet 1522 that has a stretch interference fit about the frame 1501. The frame preferably has a groove 1523 and raised edge 1524 that allows the inlet 1522 to engage with the frame.

In a further alternative form as shown in Figure 33, a seal body 1500 may be permanently attached to the frame 1501 by overmoulding or bonding.

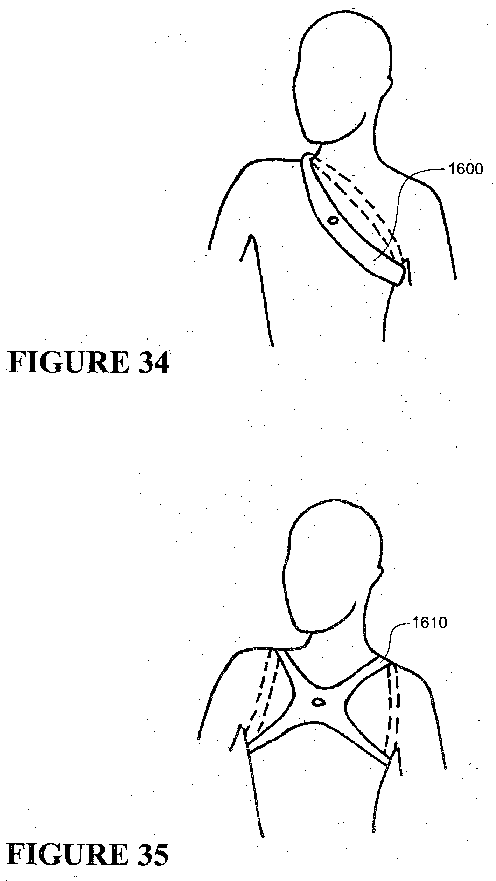

Figures 34 to 40 illustrate various ways in which the tubing (1112, see Figure 13) extending from the interface 1100 may be secured to a user. The advantage of securing the tubing to the user is to take the weight of the tubing off the interface, reducing the possibility of the interface being pulled from the user's face. Each of the braces described below is preferably made from fabric straps. It is preferred that the fabric is a breathable type material, but other appropriate fabrics may be used. In all forms detailed herein the tubing is fixed to the brace by way of a clip or pin.

In Figure 34 a brace 1600 is shown that is made from a looped strap of fabric that in use is placed about the user's head and one shoulder.

In Figure 35 an alternative brace 1610 is shown. This brace is also preferably made from fabric that is formed in a central cross across the user's chest and is braced around each of the user's arms.

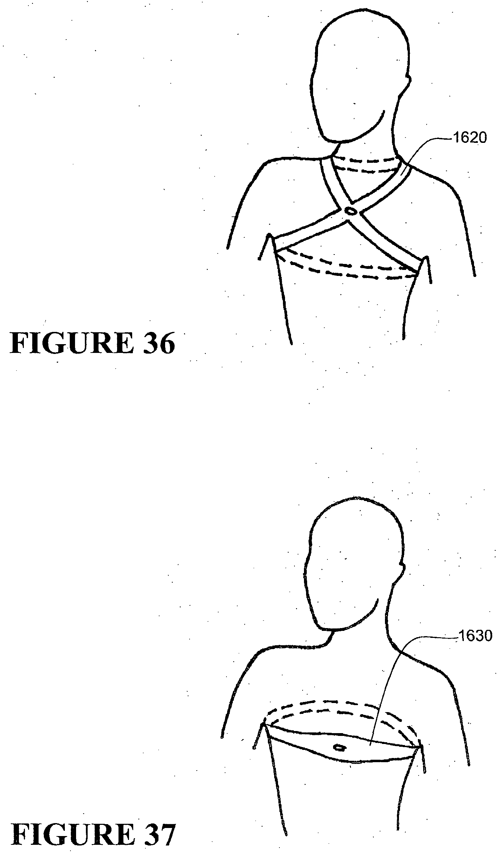

In Figure 36 a further alternative brace 1620 is shown. Here the brace 1620 has a central cross of straps across the user's chest, but it is braced across the user's neck and back.

Alternatively, as shown in Figure 37, a brace 1630 could be used to secure tubing to the user where a brace is formed from the looped strap that is extended about the user's chest and under their arms.

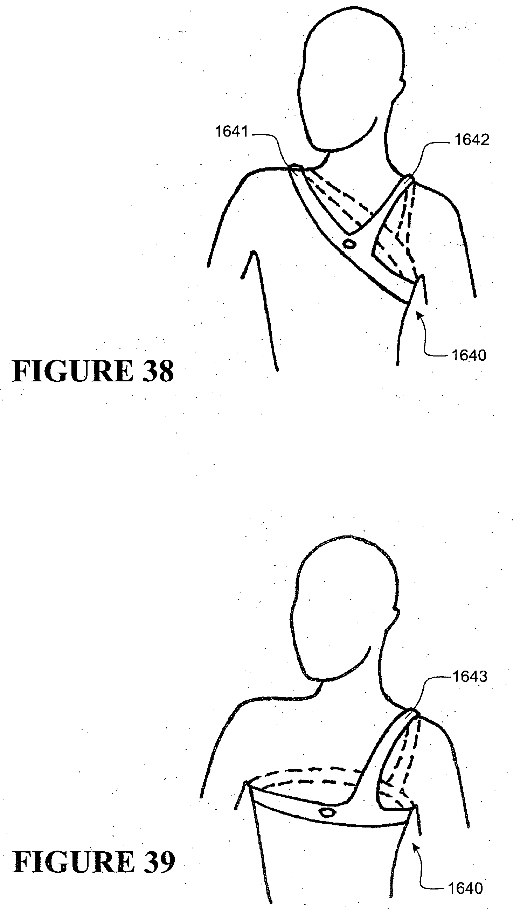

As shown in Figures 38 or 39, a brace 1640 may additionally include two shoulder straps 1641, 1642 or simply one shoulder strap 1643.

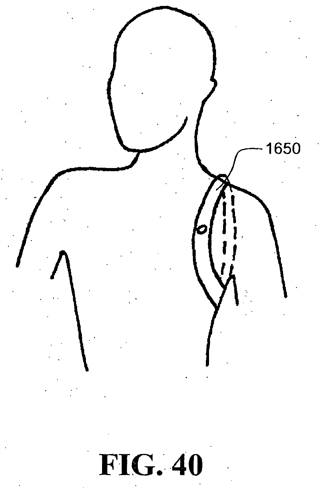

Alternatively, as shown in Figure 40, a simple brace 1650 may be used with the interface of the present invention that simply fits in use about the user's shoulder or upper arm.

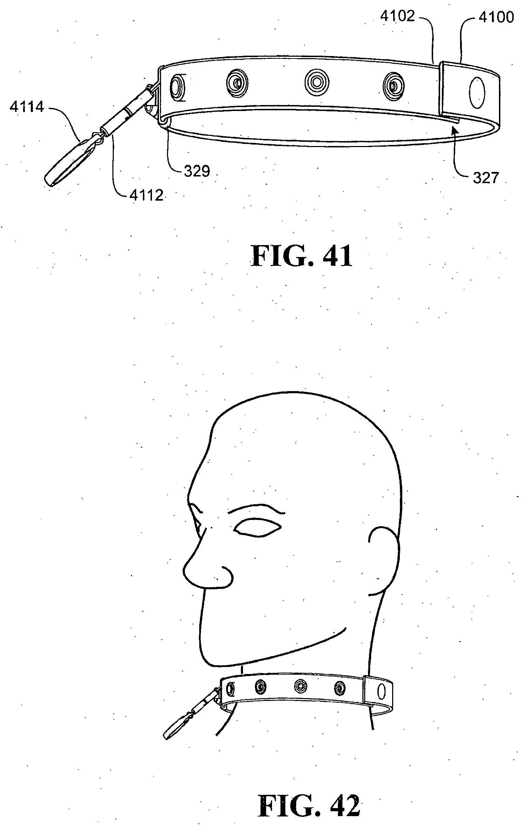

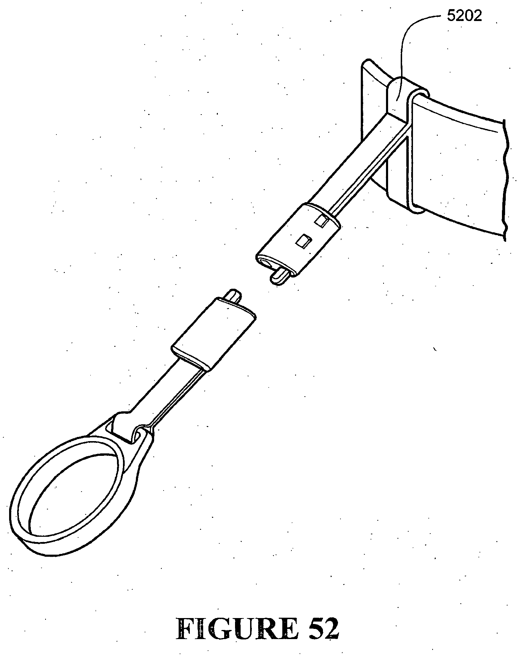

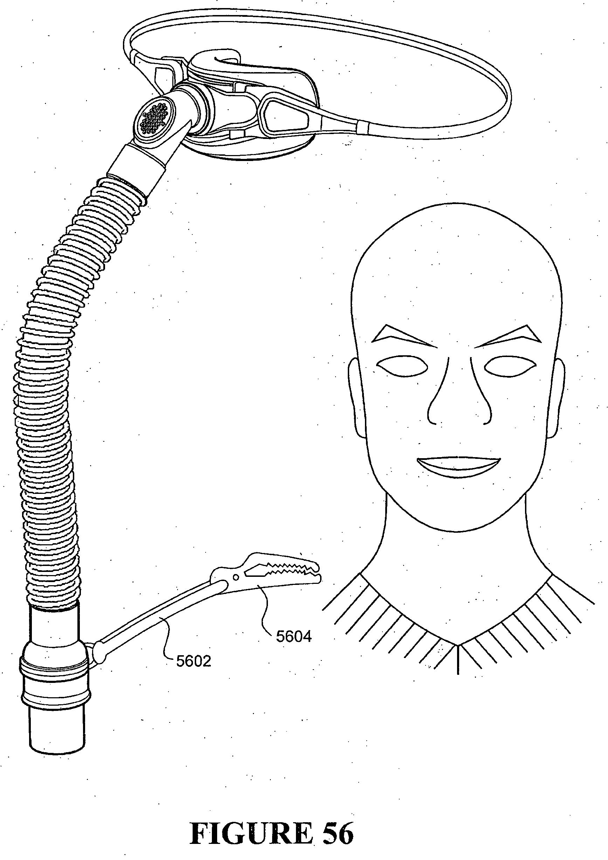

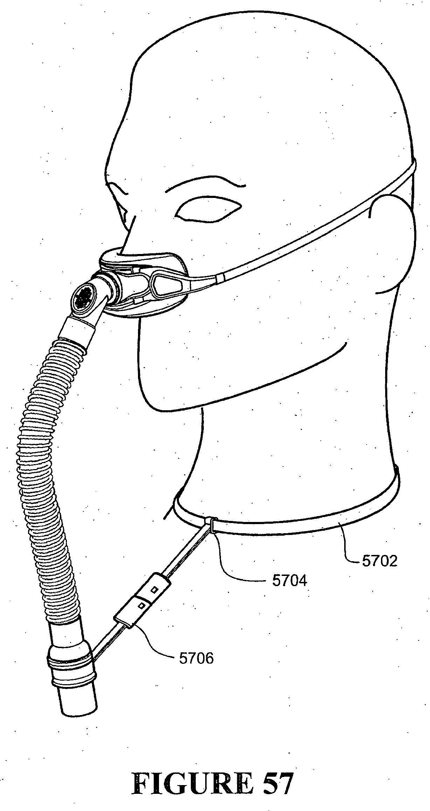

Additional tube support arrangements will be described with reference to Figures 41 to 57. Figures 41 to 51 describe a supporting collar intended to be worn around the neck of the user, and to which the tube may be supported by a tether. Figures 52 to 55 describe aspects of a tether that might be used with such a collar, or might be used with other arrangements for securing one end of the tether to the patient. Figures 56 and 57 illustrate two such arrangements for securing a tether to the patient. Figures 34 to 40 illustrate other arrangements for securing such a tether to the patient.

Figures 41 to 45 illustrate in further detail the collar previously described in broad terms in relation to Figures 1 to 3. The collar includes an adjustable connection 327 and a secondary connection 329. The adjustable connection operates between a first end of the collar 4100 and a second end 4102. The adjustable connection 327 allows the user to set the amount of overlap of the ends 4100 and 4102 to be set. Figures 41 to 45 illustrate an adjustable connection 327 in the form of a dome fastener system. One fastener portion 4104 is fixed to the first strap end 4100. A number of complementary fastener portions 4106 are provided spaced along the second strap end 4102. Engaging the fastener 4104 with one of the series of fastener portions 4106 sets the overlap of end 4100 relative to end 4102. The fastener portions 4106 may be spaced at intervals between 2cm and 5cm, preferably between 3cm to 4cm. This provides a degree of variation in the circumference of the collar in increments of between 3cm 4cm.

Preferably the outer overlapping end 4100 includes a single connector portion and the inner strap end includes a series of outwardly-facing second connector portions. According to this arrangement, no connector portion faces toward the patient neck. Accordingly, the internal surface of the collar is free of distracting projections.

The connector portions may be portions of, for example, a dome fastener of known type.

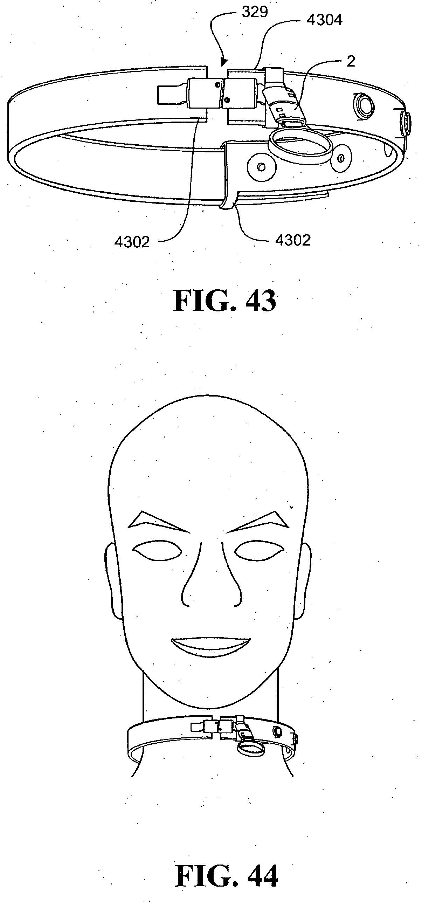

The extreme end of inner end 4102 may include an outwardly projecting loop engaging over the overlapping portion of the collar strap. This loop 4302, shown only in Figure 43, would align the free end of underlapping end 4102 with the overlapping portion of the collar when the collar is set on tighter sizes.

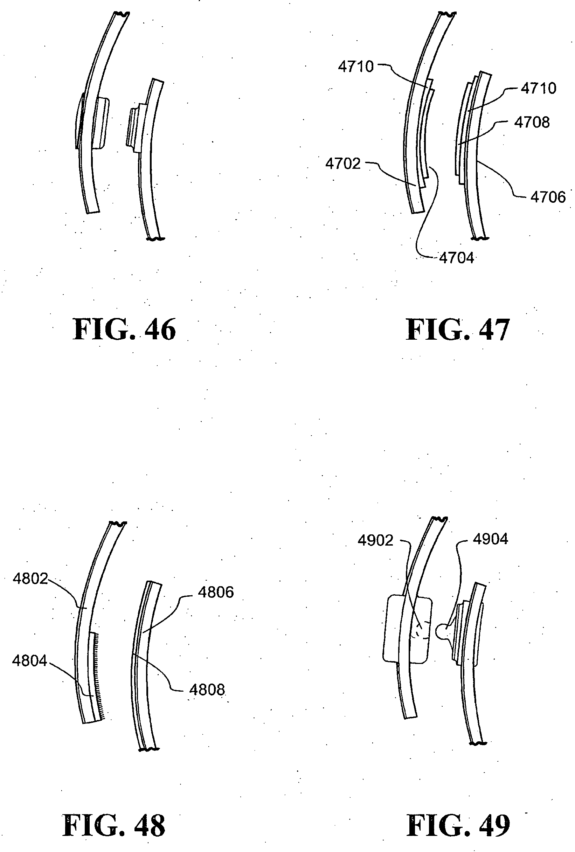

Alternative connectors for the adjustable connection are illustrated in Figures 46 to 49. The dome fastener connection is illustrated in more detail in Figure 46.

An alternative fastener using engaging magnets is illustrated in Figure 47. The outer strap end 4702 includes an inwardly-facing magnet portion 4704. The inner strap end 4706 includes an outwardly-facing magnet portion 4708. The inwardly-facing magnet portion 4704 preferably is magnetized to a first polarity facing inwards. The outwardly-facing magnet portion 4708 is preferably magnetized with a complementary polarity facing outwards. A series of outwardly-facing magnets 4708 would be spaced along the outer surface of the inner strap portion 4706.

The magnet portions may be fixed to a base portion 4710 which in turn may be fixed to the strap. For example, the magnets may be glued to a substrate material that can be stitched to the strap. Alternatively, magnets might be moulded to include holes to allow the magnets to be directly stitched to the strap.

The magnet 4704 could be replaced by a magnetic material which would be attracted by magnets 4708 but not be a magnet itself. Alternatively, magnets 4708 could be replaced by portions of a material that is magnetic but not itself a magnet. By way of example, the magnets may be ferrite or rare earth, while magnetic materials might be small sections of steel. Ferrite powder bonded with a flexible polymer may allow the magnets to be flexible while maintaining sufficient strength to secure the collar.

Figure 48 illustrates the adjustable connection being made by a hook and loop fastener system. For example, the outer end portion 4802 may include a short section 4804 of a material with projecting hooks. The inner strap end 4806 may include an outwardly-facing section 4808 covered with loops, to which the hooks may engage and disengage. Suitable hooks and loop fastener material is sold under the Velcro brand.

The outwardly facing loop material may be stitched to the collar strap, or the collar strap may be formed from a material that integrally includes the loops. The length of the loop portion 4808 is much greater than the length of the hook portion 4804 and preferably extends a length equivalent to the adjustment required to be available to the collar. For example, the loop fastener material would have a length of about 15cm along the collar strap.

Figure 49 illustrates an alternative mechanical fastener similar to the dome fastener. This type of dome fastener includes a smaller receiving aperture 4902 on the female portion and smaller projecting pins 4904 on the male portion.

Referring back to Figures 43 to 45, the collar preferably includes a second releasable connection 329 between a third end 4302 and a fourth end 4304. Thus, the overall ring of the collar is divided into two separate strap sections. Each strap section includes at one end part of the adjustable connections 327 and at the other end part of the second connection 329.

Preferably this second connection is not adjustable. This second connection 329 is intended to be engaged and disengaged at each use of the collar. The adjustable connection can be adjusted to the correct length and set, and the second connection 329 can be used to secure and release the collar.

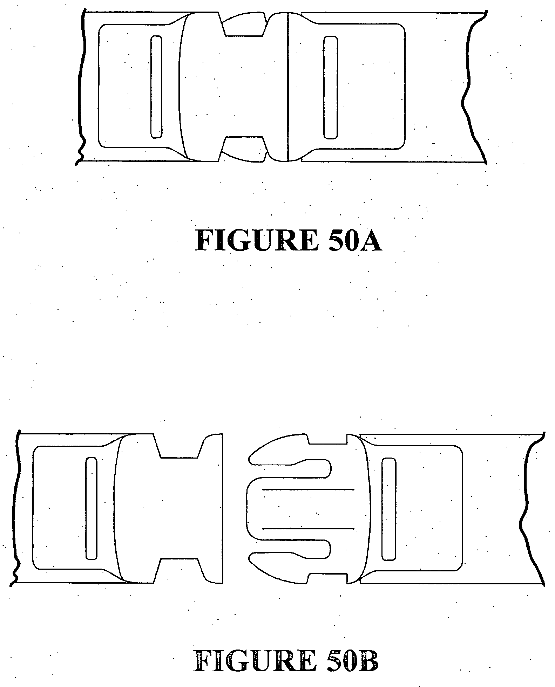

This second connection 329 may be formed by any suitable means, including the examples illustrated in Figures 46 to 49, or including a plain releasable fastening clip such as illustrated in Figures 50A and 50B (50A in the connected condition and 50B in the open condition), or a breakaway connector which releases upon application of tension in a predetermined range.

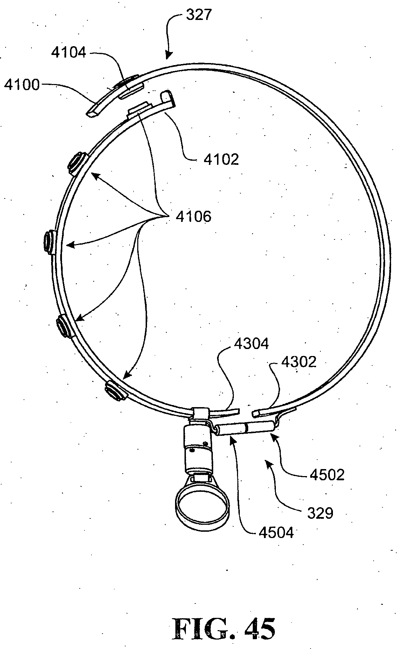

The connection 329 illustrated in Figure 45 includes a breakaway connector having a first body portion 4502 secured to strap end 4302 and a second body portion 4504 secured to fourth strap end 4304. The first and second body portions each include a projecting tang and a socket. The projecting tang of one body is complementary with the socket of the other body. The projecting tang and socket preferably have an interference fit. The amount of interference and the force required to pull the tang from the socket defines the release force for the breakaway clip.

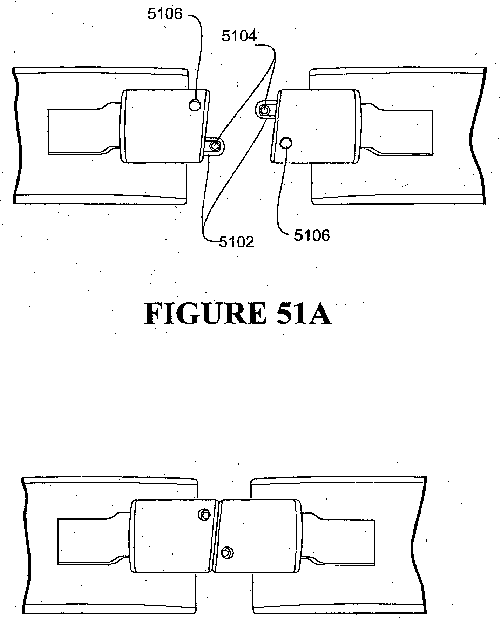

This preferred breakaway clip is illustrated in more detail in Figures 51A and 51B. In Figure 51A, the clip is illustrated in the open configuration where a tang 5102 projects from each clip body portion and each tang 5102 includes a small lateral projection 5104. The socket in each body portion of the clip includes a lateral aperture 5106. When the tang 5102 is pushed into the socket, the projection 5104 extends into the aperture 5106. The interference fit is provided by engagement of the projection 5104 in the aperture 5106. This connector is shown in its engaged condition in Figure 51B.

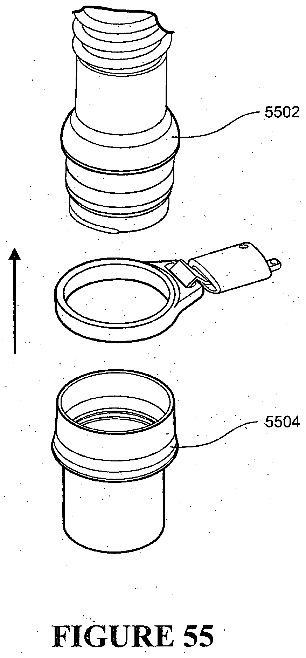

Referring again to Figures 41 to 45, a tether extends from the collar. The tether 4112 is connected with the collar at one end and to an engaging clip 4114 at its free end. The engaging clip 4114 is for connection with the supply conduit for the patient interface. The engaging clip 4114 is illustrated in greater detail in Figure 55, where an enlarged view of its fitting with the cuff of the conduit is illustrated. The preferred connector includes an open ring which fits over a sleeve portion of the cuff and is held in place between a flange 5502 of the cuff and a flange 5504 of a swivel conduit connected to the cuff.

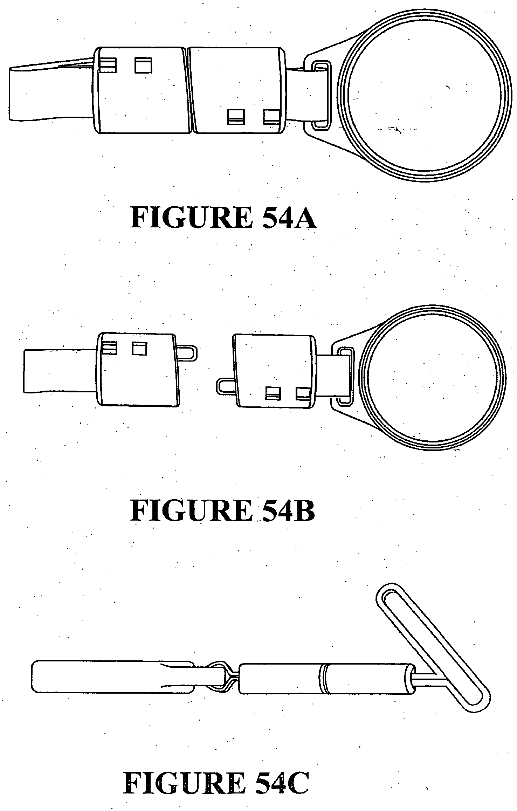

The preferred tether includes a breakaway clip at some position along its length between the connection to the collar and the conduit connector. The breakaway connector may be of the form described already with reference to Figures 51A and 51B. That form of breakaway connector is illustrated in Figures 52, 54A and 54B.

Alternatively, the breakaway connector may also include a swivel, such that the collar does not need to be correctly oriented relative to the conduit before donning the patient interface. In this case, the breakaway connector may include a socket portion 5302 and a male portion 5304, with the male portion 5304 being rotationally symmetric. For example, the male portion 5304 may include a projecting knob 5306 with an enlarged end 5308. The socket 5302 would include projecting portions or an annular projecting portion around the internal circumference adjacent the open end. The socket 5302 may be required to be made in two pieces subsequently secured together to produce this projecting lip or lips. The socket portion 5302 may be open at its other end 5310 so that the connector portion 5302 can be formed in one piece. This end may accommodate an end of a strap portion 5312 of the tether.

Alternatively, a swivel may be included at another location along the tether.

Preferably the tether is formed with a sliding connector 5202 at one end for connection over the collar. The sliding connector 5202 preferably comprises a moulded loop including straight sections either side of the web of the collar and joined by transverse sections above and below the collar edge. The loop preferably has a moderately tight fit on the collar so that once moved into a position, it tends to stay in that position but can be moved along the collar upon application of sufficient force. The loop 5202 essentially mirrors the profile of the web of the collar. A tether portion may extend from the loop 5202, preferably being integrally formed with the loop 5202. Preferably the tether portion and loop are formed from a flexible resilient material such as silicone.

Another tether portion extends from the quick release connector to the conduit in engagement clip. Again, this may be formed of any suitable material, preferably flexible and preferably a silicone material.

The tether may be of fixed or adjustable length. Preferably, the tether may be provided in multiple lengths, for selection by a patient. The tether may be of a length between 3cm and 15cm. A tether at approximately 3cm is illustrated in Figures 54A to 54B, the tether including limited, if any, strap portions. This tether is mostly made by its loop connector to the collar, by the quick release connector and by its connection to the conduit connector.

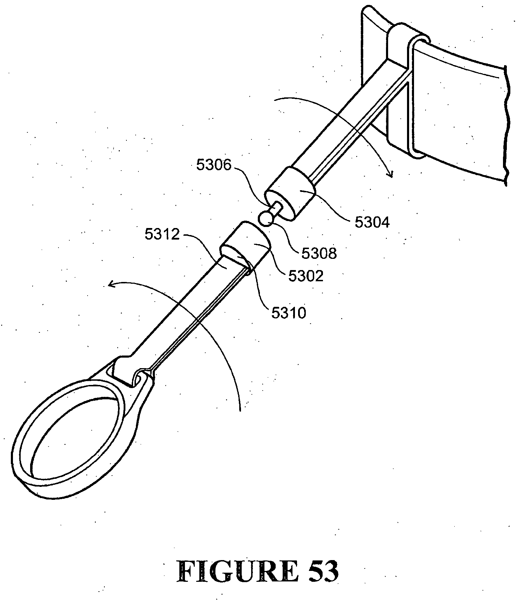

A longer tether is illustrated in Figure 52 and in Figure 53, including a substantial strap portion between the connecting loop 5202 and the quick release connector and another substantial strap portion between the quick release connector and the conduit connector. These portions of the tether could be interchanged, so that for example, the strap portion of the tether could be provided entirely to one side or other of the breakaway connector.

Figure 56 illustrates an alternative support arrangement to the use of a collar. The tether 5602 terminates in a clip 5604 instead of terminating at a connector for the collar. The clip 5604, preferably in the form of a type of peg, alligator clip or other arrangement having gripping jaws, is intended for attachment to the neckline or other convenient portion of clothing worn by the patient. Alternative, the tether may be terminated at a connector for connecting to brace structures to be worn by the patient as described earlier.

The tether may or may not include a breakaway connector.

Figure 57 illustrates another alternative for connection to clothing 5702 on the patient. This illustrates the push clip 5704 connected to the collar line of the clothing and including a breakaway connector 5706.

The preferred collar is constructed from materials comfortable to the wearer. In the simplest form, the collar might be, for example, a strap of a soft, flexible material having sufficient stiffness to hold the general collar form, sufficient strength to resist any substantial extension or stretching and comfortable inside surface facing the patient. One suitable material might be, for example, a laminated foam material such as Breathoprene, which has a foam web, faced on either side with a knitted fabric.

However, the preferred collar is more resistant to stretch than the Breathoprene material, and more breathable than the Breathoprene material. For comfort against the skin, the collar is preferably faced with a woven, knitted or braided natural fibre fabric. For example, a braided or knitted tube of cotton or bamboo yarn. To provide form to the collar, the braided or knitted tube surrounds a flexible skeleton. The flexible skeleton might comprise a series of hingedly connected frames, or a moulded flexible strap formed with an open framework. Preferably it comprises a narrow strap of plastic mesh. An example of a suitable mesh is 3MESH, manufacture by Mullter Texti Group of Germany. The open framework or mesh form allows moisture and heat to pass readily through the collar, reducing discomfort of the patient wearing the collar for long periods.

The collar strap is preferably 3cm to 6cm wide and between 3mm and 8mm thick.

One preferred simple head strap is illustrated in Figures 1 to 3. It includes a single, non-bifurcated strap terminated with a connector at either end. The strap could be permanently connected to either end of the frame, but preferably the connectors are configured to be removable from the body of the mask. Another example of a preferred headstrap is illustrated in Figure 10A to 10C, and a preferred connection clip is illustrated in Figures 63A to 63D.

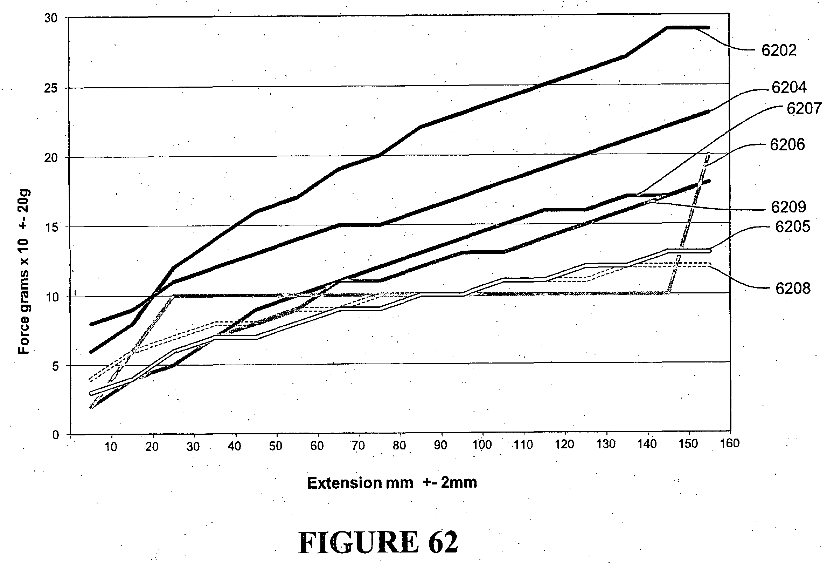

The single non-bifurcated strap preferably accommodates a substantial variation in head size without adjustment. The preferred strap has a very low stiffness, with extension of a 400mm king strap from a fully laid out but unstretched condition to a condition 1.3 times its original length requires a force not exceeding 4N, and preferably not exceeding 2N. Figure 62 is a graph illustrating the force versus extension characteristics of four sample strap materials. The preferred material comprises a knitted or braided tube nylon yarn incorporating strands of Lycra. The nylon yarn is sufficiently loosely formed that is capable of extension beyond the range required without becoming tight. The amount of Lycra strands in the yarn may be varied to vary the stiffness of the strap. An overall diameter or width of the strap is preferably less than 10mm and most preferably less than 6mm.

The end connectors of the strap may be fixed to the strap in any suitable manner. Preferably the end connectors are overmoulded to ends of the straps. Test results for a range of alternative strap materials are illustrated in Figure 62. All test results are for extensions of a length of the tested material from an "at rest" length of about 400mm.

Line 6202 shows extension test results for a knitted yarn of nylon incorporating Lycra filaments, the knitted tube having a nominal diameter of 5mm. This also is stiffer than desirable.

Line 6206 illustrates a hypothetical most desirable response determined by the inventors.

Line 6209 illustrates the response for an extended silicone hollow tube with a wall thickness of 0.25mm and an outside diameter of 3mm.

Line 6207 illustrates the response for an extended silicone hollow tube with a wall thickness of 0.25mm and an outside diameter of 6mm.

Both these silicone extensions show satisfactory characteristics.

Line 6205 illustrates the response of the preferred knitted nylon yarn incorporating Lycra filaments. This knitted tube had a nominal diameter of 4mm.

Line 6208 illustrates the response of a length of 3mm woven elastic webbing. This product exhibited similar characteristics to the preferred knitted yarn, however the elastic webbing has a tendency to catch hair and to lose elasticity.

### HEADSTRAP

The most preferred headstrap comprises a braided stretchable band. Lengths of stretchable thread are wound onto a plurality of spools. The spools of the thread are then used in a braiding machine to produce a continuous braided tube. The tube is passed over a roller or a plurality of rollers, or between rollers, to flatten the tube into a band.

The preferred headstrap has a cross-sectional dimension of approximately 6mm wide and 1.5mm thick.

According to the most preferred embodiment, the thread comprises a Lycra (elastane or sprandex) strand with a spun wrapper. The elastane strand may be, for example, 900 denier strand Lycra (elastane or spandex).

The spun wrapper may comprise at least one yarn of nylon filaments. The wrapper may comprise a primary wrapper yarn and a secondary wrapper yarn. Each yarn may comprise a spun yarn comprising a plurality of nylon filaments.

For example, each wrapper may comprise a yarn of nylon filaments.

The nylon filaments contribute the colour to the stretchable thread. For example, for a white headstrap, the nylon wrapper should comprise white nylon filaments.

In preparing a spool of the stretchy thread, preferably multiple threads (preferably three threads) of stretchy thread are wound onto the spool in parallel, so that each element in the braiding process actually comprises a bundle of three parallel threads.

Preferably, the braiding is conducted on a 16 spool braiding machine, such as a Ratera 16/80 braiding machine available from Talleres Ratera SA of Barcelona, Spain. Each spool of thread for the braiding is prepared with three parallel threads, as described above.

The braiding machine is configured (for example by setting tensions, speeds or both) to produce a suitable braid. Example settings for the Ratera 16 braiding machine speed control are A:45, B:20, C:30 and D:35.

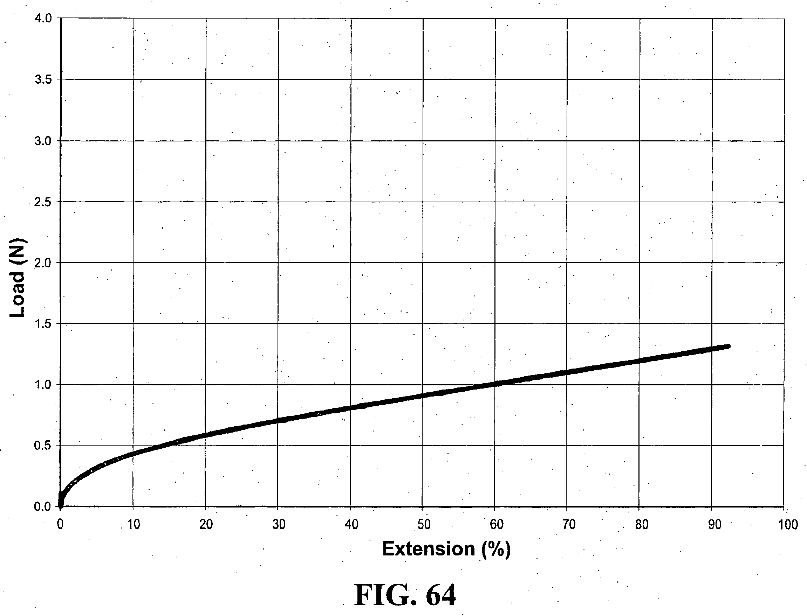

A headstrap produced in accordance with this description was tested by gradual extension, with the force at each extension recorded. The force versus extension results for a 300mm length of the prototype strap material are shown in Figure 64.

Figures 63A to 63C illustrate features of a preferred headgear strap. In particular they illustrate preferred arrangements for attaching a connector to an end of a suitable stretchy strap material. They also illustrate a preferred connector for connecting the strap to the frame of the interface.

Figures 63D to 63I illustrate another similar headgear strap which shares many of the features of the headgear strap of Figures 63A to 63C. Except where noted, the following description refers to both embodiments and reference numerals are shared.

At least one end of the strap 6304 is terminated, and preferably both ends are terminated, with a connector 6302. Once the complete interface is assembled the connectors 6302 engage the side portions 1016 of the frame.

To form the connector, the flattened tube 6306 is encapsulated by a plastic body 6308 or 6350, for example by overmoulding.

In the embodiment of Figures 63D to 63J, the band is encapsulated by a comparatively soft material such as a thermoplastic elastomer.

The plastic body 6308 may have gripping features on its outer surfaces, such as bumps or ridges 6324, or recesses 6352.

The body 6308 terminates at its other end in an end face which is preferably sized and shaped to match the end face of side portions 1016. The connector and socket are preferably formed to provide a rigid connection between the connector and the frame, such that, when engaged, the connector is a rigid extension of the side portion of the frame. According to this, the connectors contribute their length to the frame to define the location at which the soft straps depart the frame.

The preferred connection comprises a metal plug portion extending from the end face of the connector 6302, to engage in socket 1062 formed in the end face of the frame. The metal (preferably steel or titanium or similar material) plug portion has high stiffness and strength while retaining a compact form.

The plug portion may advantageously be formed from a metal wire 6310. For example the plug portion may be formed from a length of stainless steel wire.

The wire may be bent back upon itself and have both ends enclosed by the connector 6302, the wire thus forming a loop protruding from the end face of the connector. To cooperate with this loop the socket may be in the form of a slot with an overall shape to match the essentially flat prong formed by the loop.

The loop may include a limb 6316 including a protruding kink or hump. The protruding kink 6322 is adapted to engage in a shallow notch 6344 in the socket 1062. The notch 6344 may be formed along one edge surface of the slot.

The loop may be provided with a protruding end portion 6318 furthermost from the connector 6302. The slot may be provided with edge surfaces that closely match at least the lateral profile of the end portion 6318, so as to closely house the end portion with the loop engaged in the slot. This secures the connector against rotation in the plane of the loop.

The thickness of the slot preferably closely matches the thickness of the wire. This secures the connector against rotation transverse to the plane of the loop.

The loop may have a straight limb 6320, and the slot a complimentary straight edge face. These straight faces provide a sliding reference surface to align the connector with the socket during and after engagement.

Where one of the limbs of the wire loop is kinked and the other straight, and complimentary surfaces are provided in the slot, the connectors may be formed to have shapes which ensure correct orientation of the connectors relative to the socket. For example the connectors may have a form that is an obvious continuation of the form of the frame.

Where a connector and socket are provided at both ends of the strap, the connectors (and the sockets) may have reversed orientation. For example, one socket may have depression 6334 at the lower edge face and the other socket may have the depression at the upper edge face.

Alternatively the connectors may have a form or indication that ensures correct selection of the intended socket, as well as orientation relative to the selected socket.

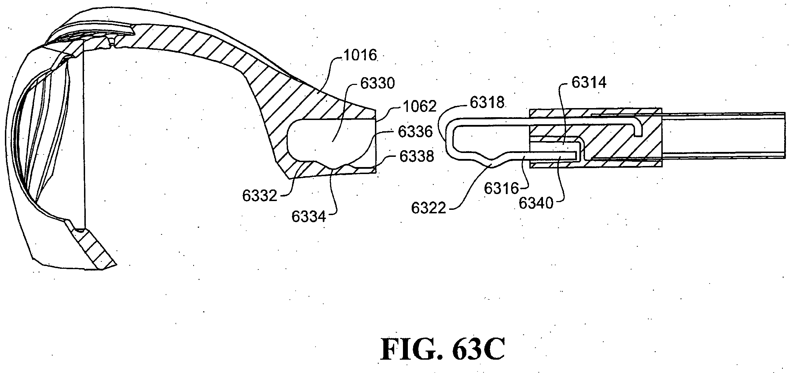

As seen in Figures 63A and 63C, one of the main limbs of the wire loop may be free to move inside a cavity 6314 of the connector body 6308. Preferably the free end 6340 is the end of the limb 6316 having the protruding kink. The small freedom to move within cavity 6314 allows the wire loop to deflect more than if the end were constrained. This deflection reduces the peak forces required (and generated in the socket) during connection and disconnection of the connector.

The cavity 6314 comprises a slot opening into the connector body 6308 from the end face of the connector body.

The other end of the wire loop may be formed to have a portion that is keyed into the material of the connector body. For example a portion of the wire may be bent into a curve and overmoulded with plastic material of the connector body. This portion may overlap inside the connector body with the encapsulated end of the strap 6304.

For forming this connector, the wire loop and the strap may be assembled with a sleeve, and the assembly be overmoulded. F or example, the sleeve (which may be plastic), may have a blind cavity that receives the end 6340 of the wire loop, and an open ended cavity that receives the other end of the wire loop (through one end of the cavity) and the end of the strap (through the other end of the cavity). The sleeve may then be overmoulded, or at least the open ended cavity holding the strap and intended fixed end of the loop may be filled. Alternatively, the sleeve may be formed in two halves, clipped together over the wire loop. The two halves may, for example, be hinged together. The halves may also clip together over the end of the band 6306 and may roughly grip the end of the band before overmoulding. For example, gripping protrusions 6360 may engage the end 6362 of the band.

According to the embodiment of Figures 63D to 63J, the soft cover material 6350 is provided by overmoulding the connector with a soft material such as a thermoplastic elastomer along a length of the very flexible floppy band 6306 beyond the sleeve. This leads to a flexible portion 6370 transitioning between a rigid portion 6372 of the connector and the very supple strap. Preferably this flexible portion is progressively more flexible moving away from the rigid portion 6372. For example, the overmoulded cover portion may taper extending away from the sleeve, indents may be provided in the cover (such as depressions 6352) or both.

On masks where the headgear strap will connect in a rigidly protruding fashion (such as in some of the embodiments of the masks described herein), the extending soft portion of the connector provides a soft buffer against impact from the protruding hard portion or prong.

Preferably the soft portion extends from 5mm to 60mm along the band or strap, most preferably from 10mm to 20mm. The soft material may be chosen from a wide range of soft plastics with consideration given to bonding with the strap material and the sleeve material.

With this connector, one of the edge surfaces of the socket 1062 may include a first portion 6332 that matches the end portion 6318 of the loop, a depression or notch 6334 that matches the protruding kink 6322, a bump 6366 which secures the kink 6322 in the notch 6334, and a recessed lead in region 6338, which allows the loop to pass well into the slot before requiring the largest connection force to push the kink 6322 past the bump 6366.

The described connector is compact, acts as an extension of the frame of the interface, and has a simple and intuitive method of connection and disconnection.

Figures 58 to 61 illustrate another patient interface incorporating a seal substantially as described earlier, and like Figures 10 to 12 includes features that may eliminate or reduce the need for additional support to the conduit. According to this embodiment, the body of the mask includes depending stabilizers 6102. A depending stabilizer is provided at each side of the mask body. Each depending stabilizer extends beyond the perimeter of the mask seal and includes a foot 6104 to engage against the upper lip of the wearer. Preferably, the stabilizer does not extend beyond the inside surface of the seal, but is spaced forwards from the inside surface of the seal, with the feet 6104 located in a position such that with the mask donned and in use symmetrically on the patient, the feet 6104 of the depending stabilizers do not contact the wearer. Each foot 6104 may include a pad 6106 of soft material such as a soft polymer or elastomer foam or a section of hollow silicone extrusion. The stabilizers be integrated with the seal rather than the frame, for example, being integrally formed as a moulded silicone body extending from adjacent the central opening to protrude beyond the lower edge of the seal. In this case, features on the mask body could secure the position of the in board ends of the stabilisers.

Each stabilizer extends in a downward direction to a region below the seal, and is intended to engage in the area of the upper lip of the patient in the area bounded by the mouth, the nose and the nasolabial folds and, preferably, not against the cheeks of the patient. Accordingly, the feet are profiled and positioned to fit within this area. Each stabilizer 6102 and arm 6108 extending from the lateral central portion 6110 of the mask body. The form of this arm, and the material of this arm may be such that the arm is rigid, or that the arm has a desired degree of flexibility. Generally, this arm should be rigid.

The purpose of the stabilizers is to reside closely spaced from the upper lip portion of the user when the interface is correctly placed and to contact the upper lip region of the user when the interface is rocked to one side or the other relative to the user's nose, for example, under the influence of the supply conduit. Gentle pressure on the foot 6104 of the stabilizer, which is laterally spaced from the centre line of the mask, preferably toward the extreme edges of the mask, supports the mask against these side forces from the conduit, stopping the mask rocking too far across the face and breaking the seal.

Furthermore, the stabilizers depend below the mask and support the mask if the weight of the conduit tends to rotate mask forward. In that case, the feet 6104 of both stabilizers will contact the user's upper lip and support the position of the mask.

The stabilizers are illustrated in preferred form as having a substantially rigid construction, but with flexible or soft pads 6106. However, to account for variations in patient geometry, these stabilizers could be a selectable appendage, with a connection arrangement to the mask allowing replacement with stabilizers of a different form. Alternatively the stabilizers could be made to be adjusted, such as by providing hinge portions capable of multiple fixed positions along the length of the stabiliser, or at the junction of the arms and feet or both. Alternatively, the arms could be formed of a malleable material which is capable of substantial yield. According to this, the arms could be flexed into a desired position by yielding of the material and stay in that position.

In the embodiment with hinging of the arms or feet, a linkage arrangement could be provided to link the movement of each of the stabilizers, or the stabilizers may be individually or collectively supported in position by a spring or springs or other resilient member.

With the addition of the stabilizers, the mask may be sufficiently secure and placed on the patient without any desire for additional support of the conduit. This in turn may allow a shorter length of flexible coupling tube 6120. Accordingly, the flexible coupling tube 6210 (which would typically be much more flexible than the main supply conduit) can be reduced in length to between 5cm and 15cm, and preferably about 10cm. In systems that include a humidified gases supply and a heated main supply conduit, this short flexible coupling tube is usually unheated. Where the coupling tube needs to be supported by a lanyard or collar, there is a minimum length generally exceeding 15cm. If the requirement for the lanyard in collar is eliminated, the shorter coupling tube is only provided for flexibility, to de-couple the relatively rigid supply conduit from the mask and facilitate freedom of movement of the wearer's head. As the coupling tube is typically unheated, the humidity of the gases carried in the tube can rainout on the cooler wall surface creating collections of water which can ultimately be blown into the user's nostrils creating discomfort. Providing a shorter tube, as allowed by the lip stabilisers, reduces the likely rainout in the conduit.

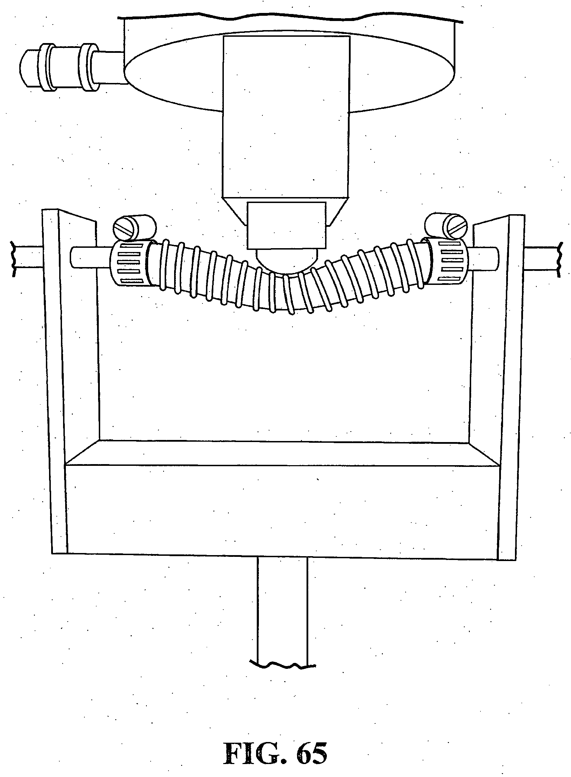

The interface configuration incorporating a single supple headstrap, a nasal seal, a low profile frame which can stabilise on the upper lip, all in a one size fits all package (preferably both the headstap and the seal) can be enhanced where the short coupling tube is especially supple. By the tube being supple, we mean that it bends easily under applied forces. For example, suitable tubes may meet the test criteria explained below with reference to Figure 65.

According to the test of Figure 65, a 150mm length of tube is clamped at each end to a cylindrical support at each end extending into the bore of the tube. This leaves approximately 130mm of the tube suspended or bridging freely between the supported ends. This bridging portion should be in a relaxed state, neither contracted or extended. A lateral force of 5N at the centre of the tube should lead to a deflection greater than 13mm.

## Claims (Claims for the following Contracting State(s): AT, BE, CH, DE, DK, ES, FR, GR, IT, LI, LU, MC, NL, SE)

1. A patient interface (101) comprising:
an inflatable nasal seal (301) comprising a face contacting side and an outward side, the nasal seal (301) being formed of a soft flexible material, and comprising a central portion to extend across the base of the nose of the wearer, and a side portion (411) extending from each end of the central portion (407), each side portion (411) extending across a side of the nose;
the face contacting side of the seal (301) being supple to conform under internal pressure to the surfaces of the nose of a wearer, including, at the side portions (411) of the seal (301), to outside surfaces of the sides of the nose;
the seal (301) including a pair of nasal locators (401) on the face contacting side;
a body connected to the seal (301), the body being formed of a material more rigid than the nasal seal (301) and comprising a nasal seal engaging portion that engages with the outward side of the seal (301), and an inlet opening; and
a lip stabiliser (6102) for engaging against an upper lip portion of the wearer.

2. The patient interface (101) according to claim 1, further comprising:
at least one strap engaging portion from which extends a loop strap (105) for securing the interface to the patient.

3. The patient interface (101) according to claim 1 or 2, wherein:
the strap (105) has a stiffness providing an extension of 150mm with a force less than 2N from a relaxed condition; and/or
the side portions (411) of the seal (301) are substantially parallel to each other and substantially normal to the central portion (407) of the seal (301).

4. The patient interface (101) according to any preceding claim, wherein the seal (301) is stiffer in the region immediately adjacent and including the nasal locators (401) than in a region surrounding this region, on the face contacting side of the seal (301).

5. The patient interface (101) according to any one of the preceding claims, wherein the pair of nasal locators (401) extend from a supple background, each nasal locator (401) including a tip, the nasal locator (401) becoming narrower moving from the background to the tip, and an opening in the tip of the nasal locator (401), the opening, and the cross-sectional profile of the tip portion of the nasal locator (401), being oval or elliptical and having a ratio of the length of the major axis to the length of the minor axis greater than 1.5.

6. The patient interface (101) according to claim 5, wherein the tip portion of the nasal locator (401) includes a lip adjacent the tip opening, the lip being thickened relative to adjacent portions of the tubular portion.

7. The patient interface (101) according to claim 5 or 6, wherein each nasal locator (401) comprises a convex base portion (1038) adjacent the background and a tubular portion (1040) extending from an apex of the convex base portion (1038), and wherein the convex base portion (1038) extends at least 60% of the way round the nasal locator (401).

8. The patient interface (101) according to any one of the preceding claims, wherein the outward side of the seal (301) comprises regions much stiffer than the supple face contacting side, the regions extending into the side portions (411) of the seal (301).

9. The patient interface (101) according to any one of the preceding claims, further comprising a freely swivelling elbow connector (313) connected to the inlet opening of the body.

10. The patient interface (101) according to any one of the preceding claims, wherein the lip stabiliser (6102) comprises one or more pads (6106) for engaging against an upper lip portion of the wearer.

11. The patient interface (101) according to any one of the preceding claims, wherein the lip stabiliser (6102) includes two depending legs (6108), spaced apart at either lateral region of the seal (301).

12. The patient interface (101) according to any one of the preceding claims, wherein the lip stabiliser (6102) depends from the body (303), and extends beyond a lower edge of the seal (301).

13. The patient interface (101) according to any one of claims 1-11, wherein the lip stabiliser (6102) is integrally formed with the seal (301).

14. The patient interface (101) according to any of claims 2 to 13, wherein the strap (105) is a single loop, non-bifurcated headstrap; and/or the strap (105) engages the body (303) with a releasable connector (321) at either end.

15. The patient interface (101) according to any one of the preceding claims, further comprising:
a flexible gases supply tube (315) coupled to the inlet opening of the body (303) for free swivelling movement, the tube (315) being less than 200mm long; and/or
a mouth covering extension (1203) having an outlet (1304) directed toward the user's mouth such that the patient interface (101) enables, in use, gases to be delivered simultaneously to the user's mouth through the outlet (1304) and nasal passages through the nasal seal (301).

## Claims (Claims for the following Contracting State(s): GB)

1. A patient interface (101) comprising:
an inflatable nasal seal (301) comprising a face contacting side and an outward side, the nasal seal (301) being formed of a soft flexible material, and comprising a central portion to extend across the base of the nose of the wearer, and a side portion (411) extending from each end of the central portion (407), each side portion (411) extending across a side of the nose;
the face contacting side of the seal (301) being supple to conform under internal pressure to the surfaces of the nose of a wearer, including, at the side portions (411) of the seal (301), to outside surfaces of the sides of the nose;
the seal (301) including a pair of nasal locators (401) on the face contacting side;
a body connected to the seal (301), the body being formed of a material more rigid than the nasal seal (301) and comprising a nasal seal engaging portion that engages with the outward side of the seal (301), and an inlet opening; and
a lip stabiliser (6102) for engaging against an upper lip portion of the wearer.

2. The patient interface (101) according to claim 1, further comprising:
at least one strap engaging portion from which extends a loop strap (105) for securing the interface to the patient.

3. The patient interface (101) according to claim 1 or 2, wherein:
the side portions (411) of the seal (301) are substantially parallel to each other and substantially normal to the central portion (407) of the seal (301).

4. The patient interface (101) according to any preceding claim, wherein the seal (301) is stiffer in the region immediately adjacent and including the nasal locators (401) than in a region surrounding this region, on the face contacting side of the seal (301).

5. The patient interface (101) according to any one of the preceding claims, wherein the pair of nasal locators (401) extend from a supple background, each nasal locator (401) including a tip, the nasal locator (401) becoming narrower moving from the background to the tip, and an opening in the tip of the nasal locator (401), the opening, and the cross-sectional profile of the tip portion of the nasal locator (401), being oval or elliptical and having a ratio of the length of the major axis to the length of the minor axis greater than 1.5.

6. The patient interface (101) according to claim 5, wherein the tip portion of the nasal locator (401) includes a lip adjacent the tip opening, the lip being thickened relative to adjacent portions of the tubular portion.

7. The patient interface (101) according to claim 5 or 6, wherein each nasal locator (401) comprises a convex base portion (1038) adjacent the background and a tubular portion (1040) extending from an apex of the convex base portion (1038), and wherein the convex base portion (1038) extends at least 60% of the way round the nasal locator (401).

8. The patient interface (101) according to any one of the preceding claims, wherein the outward side of the seal (301) comprises regions much stiffer than the supple face contacting side, the regions extending into the side portions (411) of the seal (301).

9. The patient interface (101) according to any one of the preceding claims, further comprising a freely swivelling elbow connector (313) connected to the inlet opening of the body.

10. The patient interface (101) according to any one of the preceding claims, wherein the lip stabiliser (6102) comprises one or more pads (6106) for engaging against an upper lip portion of the wearer.

11. The patient interface (101) according to any one of the preceding claims, wherein the lip stabiliser (6102) includes two depending legs (6108), spaced apart at either lateral region of the seal (301).

12. The patient interface (101) according to any one of the preceding claims, wherein the lip stabiliser (6102) depends from the body (303), and extends beyond a lower edge of the seal (301).

13. The patient interface (101) according to any one of claims 1-11, wherein the lip stabiliser (6102) is integrally formed with the seal (301).

14. The patient interface (101) according to any of claims 2 to 13, wherein the strap (105) is a single loop, non-bifurcated headstrap; and/or the strap (105) engages the body (303) with a releasable connector (321) at either end.

15. The patient interface (101) according to any one of the preceding claims, further comprising:
a flexible gases supply tube (315) coupled to the inlet opening of the body (303) for free swivelling movement, the tube (315) being less than 200mm long; and/or
a mouth covering extension (1203) having an outlet (1304) directed toward the user's mouth such that the patient interface (101) enables, in use, gases to be delivered simultaneously to the user's mouth through the outlet (1304) and nasal passages through the nasal seal (301).

## Patentansprüche (Patentansprüche für folgende(n) Vertragsstaat(en): AT, BE, CH, DE, DK, ES, FR, GR, IT, LI, LU, MC, NL, SE)

1. Patientenschnittstelle (101), umfassend:
eine aufblasbare Nasendichtung (301) umfassend eine Gesichtskontaktseite und eine Außenseite, wobei die Nasendichtung (301) aus einem weichen flexiblen Material geformt ist, und umfassend einen mittleren Abschnitt, der sich über die Nasenbasis des Trägers erstreckt, und einen Seitenabschnitt (411), der sich von jedem Ende des mittleren Abschnitts (407) aus erstreckt, wobei sich jeder Seitenabschnitt (411) über eine Seite der Nase erstreckt;
wobei die Gesichtskontaktseite der Dichtung (301) biegsam ist, damit sie sich unter internem Druck an die Oberflächen der Nase eines Trägers, einschließlich an den Seitenabschnitten (411) der Dichtung (301), an Außenflächen der Nasenseiten anpassen kann;
wobei die Dichtung (301) ein Nasenlokalisatorpaar (401) auf der Gesichtskontaktseite beinhaltet;
einen Körper, der mit der Dichtung (301) verbunden ist, wobei der Körper aus einem Material geformt ist, das starrer ist als die Nasendichtung (301), und umfassend einen Nasendichtungseingriffsabschnitt, der die Außenseite der Dichtung (301) in Eingriff nimmt, und eine Einlassöffnung; und
eine Lippenstütze (6102) zum Eingriff gegen einen Oberlippenabschnitt des Trägers.

2. Patientenschnittstelle (101) nach Anspruch 1, ferner umfassend:
mindestens einen Gurteingriffsabschnitt, von dem sich ein Schlaufengurt (105) zur Fixierung der Schnittstelle am Patienten erstreckt.

3. Patientenschnittstelle (101) nach Anspruch 1 oder 2, worin:
der Gurt (105) eine Steifheit aufweist, wodurch eine Ausdehnung von 150 mm mit einer Kraft von weniger als 2 N aus einem entspannten Zustand geboten wird; und/oder
die Seitenabschnitte (411) der Dichtung (301) im Wesentlichen parallel zueinander und im Wesentlichen senkrecht zum mittleren Abschnitt (407) der Dichtung (301) sind.

4. Patientenschnittstelle (101) nach einem vorhergehenden Anspruch, worin die Dichtung (301) in der Region, die unmittelbar neben den Nasenlokalisatoren (401) liegt und diese einschließt, steifer ist als in einer diese Region umgebenden Region, auf der Gesichtskontaktseite der Dichtung (301).

5. Patientenschnittstelle (101) nach einem der vorhergehenden Ansprüche, worin sich das Nasenlokalisatorpaar (401) von einem biegsamen Hintergrund aus erstreckt, wobei jeder Nasenlokalisator (401) eine Spitze, wobei der Nasenlokalisator (401) bei Bewegung vom Hintergrund zur Spitze schmaler wird, und eine Öffnung in der Spitze des Nasenlokalisators (401) beinhaltet, wobei die Öffnung, und das Querschnittsprofil des Spitzenabschnitts des Nasenlokalisators (401), oval oder elliptisch sind und dabei ein Verhältnis der Länge der Hauptachse zur Länge der Nebenachse von mehr als 1,5 vorliegt.

6. Patientenschnittstelle (101) nach Anspruch 5, worin der Spitzenabschnitt des Nasenlokalisators (401) eine an der Spitzenöffnung anliegende Lippe beinhaltet, wobei die Lippe relativ zu anliegenden Abschnitten des Schlauchabschnitts verdickt ist.

7. Patientenschnittstelle (101) nach Anspruch 5 oder 6, worin jeder Nasenlokalisator (401) einen konvexen Basisabschnitt (1038), der neben dem Hintergrund liegt, und einen Schlauchabschnitt (1040) umfasst, der sich von einem Scheitel des konvexen Basisabschnitts (1038) erstreckt, und worin sich der konvexe Basisabschnitt (1038) mindestens 60 % um den Nasenlokalisator (401) herum erstreckt.

8. Patientenschnittstelle (101) nach einem der vorhergehenden Ansprüche, worin die Außenseite der Dichtung (301) Regionen umfasst, die viel steifer sind als die biegsame Gesichtskontaktseite, wobei sich die Regionen bis in die Seitenabschnitte (411) der Dichtung (301) erstrecken.

9. Patientenschnittstelle (101) nach einem der vorhergehenden Ansprüche, ferner umfassend einen frei schwenkbaren Ellbogenverbinder (313), der mit der Einlassöffnung des Körpers verbunden ist.

10. Patientenschnittstelle (101) nach einem der vorhergehenden Ansprüche, worin die Lippenstütze (6102) ein oder mehr Polster (6106) zum Eingriff gegen einen Oberlippenabschnitt des Trägers umfasst.

11. Patientenschnittstelle (101) nach einem der vorhergehenden Ansprüche, worin die Lippenstütze (6102) zwei herabhängende Schenkel (6108) beinhaltet, die in jeder seitlichen Region der Dichtung (301) voneinander beabstandet sind.

12. Patientenschnittstelle (101) nach einem der vorhergehenden Ansprüche, worin die Lippenstütze (6102) vom Körper (303) herabhängt und sich über eine Unterkante der Dichtung (301) hinaus erstreckt.

13. Patientenschnittstelle (101) nach einem der Ansprüche 1-11, worin die Lippenstütze (6102) integral mit der Dichtung (301) geformt ist.

14. Patientenschnittstelle (101) nach einem der Ansprüche 2 bis 13, worin der Gurt (105) ein einschlaufiger, nicht gegabelter Kopfgurt ist; und/oder der Gurt (105) den Körper (303) mit einem lösbaren Verbinder (321) an jedem Ende in Eingriff nimmt.

15. Patientenschnittstelle (101) nach einem der vorhergehenden Ansprüche, ferner umfassend:
einen flexiblen Gaszuführungsschlauch (315), der mit der Einlassöffnung des Körpers (303) zur freien Schwenkbewegung verbunden ist, wobei der Schlauch (315) weniger als 200 mm lang ist; und/oder
eine den Mund abdeckende Verlängerung (1203) mit einem Auslass (1304), der zum Mund des Benutzers hin gerichtet ist, so dass die Patientenschnittstelle (101) es bei Gebrauch ermöglicht, dass Gase dem Mund des Benutzers durch den Auslass (1304) und Nasenpassagen durch die Nasendichtung (301) gleichzeitig zugeführt werden.

## Patentansprüche (Patentansprüche für folgende(n) Vertragsstaat(en): GB)

1. Patientenschnittstelle (101), umfassend:
eine aufblasbare Nasendichtung (301) umfassend eine Gesichtskontaktseite und eine Außenseite, wobei die Nasendichtung (301) aus einem weichen flexiblen Material geformt ist, und umfassend einen mittleren Abschnitt, der sich über die Nasenbasis des Trägers erstreckt, und einen Seitenabschnitt (411), der sich von jedem Ende des mittleren Abschnitts (407) aus erstreckt, wobei sich jeder Seitenabschnitt (411) über eine Seite der Nase erstreckt;
wobei die Gesichtskontaktseite der Dichtung (301) biegsam ist, damit sie sich unter internem Druck an die Oberflächen der Nase eines Trägers, einschließlich an den Seitenabschnitten (411) der Dichtung (301), an Außenflächen der Nasenseiten anpassen kann;
wobei die Dichtung (301) ein Nasenlokalisatorpaar (401) auf der Gesichtskontaktseite beinhaltet;
einen Körper, der mit der Dichtung (301) verbunden ist, wobei der Körper aus einem Material geformt ist, das starrer ist als die Nasendichtung (301), und umfassend einen Nasendichtungseingriffsabschnitt, der die Außenseite der Dichtung (301) in Eingriff nimmt, und eine Einlassöffnung; und
eine Lippenstütze (6102) zum Eingriff gegen einen Oberlippenabschnitt des Trägers.

2. Patientenschnittstelle (101) nach Anspruch 1, ferner umfassend:
mindestens einen Gurteingriffsabschnitt, von dem sich ein Schlaufengurt (105) zur Fixierung der Schnittstelle am Patienten erstreckt.

3. Patientenschnittstelle (101) nach Anspruch 1 oder 2, worin:
die Seitenabschnitte (411) der Dichtung (301) im Wesentlichen parallel zueinander und im Wesentlichen senkrecht zum mittleren Abschnitt (407) der Dichtung (301) sind.

4. Patientenschnittstelle (101) nach einem vorhergehenden Anspruch, worin die Dichtung (301) in der Region, die unmittelbar neben den Nasenlokalisatoren (401) liegt und diese einschließt, steifer ist als in einer diese Region umgebenden Region, auf der Gesichtskontaktseite der Dichtung (301).

5. Patientenschnittstelle (101) nach einem der vorhergehenden Ansprüche, worin sich das Nasenlokalisatorpaar (401) von einem biegsamen Hintergrund aus erstreckt, wobei jeder Nasenlokalisator (401) eine Spitze, wobei der Nasenlokalisator (401) bei Bewegung vom Hintergrund zur Spitze schmaler wird, und eine Öffnung in der Spitze des Nasenlokalisators (401) beinhaltet, wobei die Öffnung, und das Querschnittsprofil des Spitzenabschnitts des Nasenlokalisators (401), oval oder elliptisch sind und dabei ein Verhältnis der Länge der Hauptachse zur Länge der Nebenachse von mehr als 1,5 vorliegt.

6. Patientenschnittstelle (101) nach Anspruch 5, worin der Spitzenabschnitt des Nasenlokalisators (401) eine an der Spitzenöffnung anliegende Lippe beinhaltet, wobei die Lippe relativ zu anliegenden Abschnitten des Schlauchabschnitts verdickt ist.

7. Patientenschnittstelle (101) nach Anspruch 5 oder 6, worin jeder Nasenlokalisator (401) einen konvexen Basisabschnitt (1038), der neben dem Hintergrund liegt, und einen Schlauchabschnitt (1040) umfasst, der sich von einem Scheitel des konvexen Basisabschnitts (1038) erstreckt, und worin sich der konvexe Basisabschnitt (1038) mindestens 60 % um den Nasenlokalisator (401) herum erstreckt.

8. Patientenschnittstelle (101) nach einem der vorhergehenden Ansprüche, worin die Außenseite der Dichtung (301) Regionen umfasst, die viel steifer sind als die biegsame Gesichtskontaktseite, wobei sich die Regionen bis in die Seitenabschnitte (411) der Dichtung (301) erstrecken.

9. Patientenschnittstelle (101) nach einem der vorhergehenden Ansprüche, ferner umfassend einen frei schwenkbaren Ellbogenverbinder (313), der mit der Einlassöffnung des Körpers verbunden ist.

10. Patientenschnittstelle (101) nach einem der vorhergehenden Ansprüche, worin die Lippenstütze (6102) ein oder mehr Polster (6106) zum Eingriff gegen einen Oberlippenabschnitt des Trägers umfasst.

11. Patientenschnittstelle (101) nach einem der vorhergehenden Ansprüche, worin die Lippenstütze (6102) zwei herabhängende Schenkel (6108) beinhaltet, die in jeder seitlichen Region der Dichtung (301) voneinander beabstandet sind.

12. Patientenschnittstelle (101) nach einem der vorhergehenden Ansprüche, worin die Lippenstütze (6102) vom Körper (303) herabhängt und sich über eine Unterkante der Dichtung (301) hinaus erstreckt.

13. Patientenschnittstelle (101) nach einem der Ansprüche 1-11, worin die Lippenstütze (6102) integral mit der Dichtung (301) geformt ist.

14. Patientenschnittstelle (101) nach einem der Ansprüche 2 bis 13, worin der Gurt (105) ein einschlaufiger, nicht gegabelter Kopfgurt ist; und/oder der Gurt (105) den Körper (303) mit einem lösbaren Verbinder (321) an jedem Ende in Eingriff nimmt.

15. Patientenschnittstelle (101) nach einem der vorhergehenden Ansprüche, ferner umfassend:
einen flexiblen Gaszuführungsschlauch (315), der mit der Einlassöffnung des Körpers (303) zur freien Schwenkbewegung verbunden ist, wobei der Schlauch (315) weniger als 200 mm lang ist; und/oder
eine den Mund abdeckende Verlängerung (1203) mit einem Auslass (1304), der zum Mund des Benutzers hin gerichtet ist, so dass die Patientenschnittstelle (101) es bei Gebrauch ermöglicht, dass Gase dem Mund des Benutzers durch den Auslass (1304) und Nasenpassagen durch die Nasendichtung (301) gleichzeitig zugeführt werden.

## Revendications (Revendications pour l'(les) Etat(s) contractant(s) suivant(s): AT, BE, CH, DE, DK, ES, FR, GR, IT, LI, LU, MC, NL, SE)

1. Interface de patient (101) comprenant :
un joint nasal gonflable (301) comprenant un côté de contact avec le visage et un côté extérieur, le joint nasal (301) étant formé d'un matériau mou flexible et comprenant une partie centrale qui s'étend sur la base du nez du porteur, et une partie latérale (411) qui s'étend à partir de chaque extrémité de la partie centrale (407), chaque partie latérale (411) s'étendant sur un côté du nez ;
le côté de contact avec le visage du joint (301) étant souple pour épouser, sous une pression interne, les surfaces du nez d'un porteur, y compris, au niveau des parties latérales (411) du joint (301), les surfaces extérieures des côtés du nez ;
le joint (301) comprenant une paire de repères nasaux (401) sur le côté de contact avec le visage ;
un corps connecté au joint (301), le corps étant constitué d'un matériau plus rigide que le joint nasal (301) et comprenant une partie de réception de joint nasal qui reçoit le côté extérieur du joint (301), et une ouverture intérieure ; et
un stabilisateur de lèvre (6102) pour venir au contact d'une partie de lèvre supérieure du porteur.

2. Interface de patient (101) selon la revendication 1, comprenant en outre :
au moins une partie de solidarisation de sangle à partir de laquelle s'étend une sangle (105) en boucle pour fixer l'interface au patient.

3. Interface de patient (101) selon la revendication 1 ou 2, dans laquelle :
la sangle (105) a une rigidité permettant une extension de 150 mm avec une force inférieure à 2 N à partir d'un état relâché ; et/ou
les parties latérales (411) du joint (301) sont sensiblement parallèles l'une par rapport à l'autre et sont pratiquement normales à la partie centrale (407) du joint (301).

4. Interface de patient (101) selon l'une quelconque des revendications précédentes, dans laquelle le joint (301) est plus rigide dans la région immédiatement adjacente et comprenant les repères nasaux (401) que dans une région entourant cette région, sur le côté de contact avec le visage du joint (301).

5. Interface de patient (101) selon l'une quelconque des revendications précédentes, dans laquelle la paire de repères nasaux (401) s'étend à partir d'un fond souple, chaque repère nasal (401) comprenant une pointe, le repère nasal (401) devenant plus étroit du fond vers la pointe, et une ouverture dans la pointe du repère nasal (401), l'ouverture, et le profil transversal de la partie pointe du repère nasal (401), étant ovale ou elliptique et ayant un rapport entre la longueur de l'axe principal et la longueur de l'axe mineur supérieur à 1,5.

6. Interface de patient (101) selon la revendication 5, dans laquelle la partie pointe du repère nasal (401) comprend une lèvre adjacente à l'ouverture de lèvre, la lèvre étant épaissie par rapport à des parties adjacentes de la partie tubulaire.

7. Interface de patient (101) selon la revendication 5 ou 6, dans lequel chaque repère nasal (401) comprend une partie de base convexe (1038) adjacente au fond et une partie tubulaire (1040) s'étendant à partir d'un sommet de la partie de base convexe (1038), et la partie de base convexe (1038) s'étendant sur au moins 60 % autour du repère nasal (401).

8. Interface de patient (101) selon l'une quelconque des revendications précédentes, dans laquelle le côté extérieur du joint (301) comprend des régions plus rigides que le côté souple de contact avec le visage, les régions s'étendant dans les parties latérales (411) du joint (301).

9. Interface de patient (101) selon l'une quelconque des revendications précédentes, comprenant en outre un connecteur coudé librement orientable (313) connecté à l'ouverture d'entrée du corps.

10. Interface de patient (101) selon l'une quelconque des revendications précédentes, dans laquelle le stabilisateur de lèvre (6102) comprend un ou plusieurs rembourrages (6106) pour venir au contact d'une partie de lèvre supérieure du porteur.

11. Interface de patient (101) selon l'une quelconque des revendications précédentes, dans laquelle le stabilisateur de lèvre (6102) comprend deux pattes dépendantes (6108), espacées au niveau de chaque région latérale du joint (301).

12. Interface de patient (101) selon l'une quelconque des revendications précédentes, dans laquelle le stabilisateur de lèvre (6102) dépend du corps (303) et s'étend au-delà d'un bord inférieur du joint (301).

13. Interface de patient (101) selon l'une quelconque des revendications 1 à 11, dans laquelle le stabilisateur de lèvre (6102) fait partie intégrante du joint (301).

14. Interface de patient (101) selon l'une quelconque des revendications 2 à 13, dans lequel la sangle (105) est un serre-tête non bifurqué à boucle unique ; et/ou la sangle (105) est solidarisée au corps (303) au moyen d'un connecteur amovible (321) à chaque extrémité.

15. Interface de patient (101) selon l'une quelconque des revendications précédentes, comprenant en outre :
un tube flexible d'alimentation en gaz (315) accouplé à l'ouverture d'entrée du corps (303) en vue d'un mouvement orientable libre, le tube (315) mesurant moins de 200 mm de long ; et/ou
une extension de couverture de bouche (1203) comportant une sortie (1304) directement vers la bouche de l'utilisateur, de sorte que l'interface de patient (101) permette, lors de l'utilisation, d'administrer simultanément des gaz dans la bouche du patient par la sortie (1304) et dans des passages nasaux par le joint nasal (301).

## Revendications (Revendications pour l'(les) Etat(s) contractant(s) suivant(s): GB)

1. Interface de patient (101) comprenant :
un joint nasal gonflable (301) comprenant un côté de contact avec le visage et un côté extérieur, le joint nasal (301) étant formé d'un matériau mou flexible et comprenant une partie centrale qui s'étend sur la base du nez du porteur, et une partie latérale (411) qui s'étend à partir de chaque extrémité de la partie centrale (407), chaque partie latérale (411) s'étendant sur un côté du nez ;
le côté de contact avec le visage du joint (301) étant souple pour épouser, sous une pression interne, les surfaces du nez d'un porteur, y compris, au niveau des parties latérales (411) du joint (301), les surfaces extérieures des côtés du nez ;
le joint (301) comprenant une paire de repères nasaux (401) sur le côté de contact avec le visage ;
un corps connecté au joint (301), le corps étant constitué d'un matériau plus rigide que le joint nasal (301) et comprenant une partie de réception de joint nasal qui reçoit le côté extérieur du joint (301), et une ouverture intérieure ; et
un stabilisateur de lèvre (6102) pour venir au contact d'une partie de lèvre supérieure du porteur.

2. Interface de patient (101) selon la revendication 1, comprenant en outre :
au moins une partie de solidarisation de sangle à partir de laquelle s'étend une sangle (105) en boucle pour fixer l'interface au patient.

3. Interface de patient (101) selon la revendication 1 ou 2, dans laquelle :
les parties latérales (411) du joint (301) sont sensiblement parallèles l'une par rapport à l'autre et sont pratiquement normales à la partie centrale (407) du joint (301).

4. Interface de patient (101) selon l'une quelconque des revendications précédentes, dans laquelle le joint (301) est plus rigide dans la région immédiatement adjacente et comprenant les repères nasaux (401) que dans une région entourant cette région, sur le côté de contact avec le visage du joint (301).

5. Interface de patient (101) selon l'une quelconque des revendications précédentes, dans laquelle la paire de repères nasaux (401) s'étend à partir d'un fond souple, chaque repère nasal (401) comprenant une pointe, le repère nasal (401) devenant plus étroit du fond vers la pointe, et une ouverture dans la pointe du repère nasal (401), l'ouverture, et le profil transversal de la partie pointe du repère nasal (401), étant ovale ou elliptique et ayant un rapport entre la longueur de l'axe principal et la longueur de l'axe mineur supérieur à 1,5.

6. Interface de patient (101) selon la revendication 5, dans laquelle la partie pointe du repère nasal (401) comprend une lèvre adjacente à l'ouverture de lèvre, la lèvre étant épaissie par rapport à des parties adjacentes de la partie tubulaire.

7. Interface de patient (101) selon la revendication 5 ou 6, dans lequel chaque repère nasal (401) comprend une partie de base convexe (1038) adjacente au fond et une partie tubulaire (1040) s'étendant à partir d'un sommet de la partie de base convexe (1038), et la partie de base convexe (1038) s'étendant sur au moins 60 % autour du repère nasal (401).

8. Interface de patient (101) selon l'une quelconque des revendications précédentes, dans laquelle le côté extérieur du joint (301) comprend des régions plus rigides que le côté souple de contact avec le visage, les régions s'étendant dans les parties latérales (411) du joint (301).

9. Interface de patient (101) selon l'une quelconque des revendications précédentes, comprenant en outre un connecteur coudé librement orientable (313) connecté à l'ouverture d'entrée du corps.

10. Interface de patient (101) selon l'une quelconque des revendications précédentes, dans laquelle le stabilisateur de lèvre (6102) comprend un ou plusieurs rembourrages (6106) pour venir au contact d'une partie de lèvre supérieure du porteur.

11. Interface de patient (101) selon l'une quelconque des revendications précédentes, dans laquelle le stabilisateur de lèvre (6102) comprend deux pattes dépendantes (6108), espacées au niveau de chaque région latérale du joint (301).

12. Interface de patient (101) selon l'une quelconque des revendications précédentes, dans laquelle le stabilisateur de lèvre (6102) dépend du corps (303) et s'étend au-delà d'un bord inférieur du joint (301).

13. Interface de patient (101) selon l'une quelconque des revendications 1 à 11, dans laquelle le stabilisateur de lèvre (6102) fait partie intégrante du joint (301).

14. Interface de patient (101) selon l'une quelconque des revendications 2 à 13, dans lequel la sangle (105) est un serre-tête non bifurqué à boucle unique ; et/ou la sangle (105) est solidarisée au corps (303) au moyen d'un connecteur amovible (321) à chaque extrémité.

15. Interface de patient (101) selon l'une quelconque des revendications précédentes, comprenant en outre :
un tube flexible d'alimentation en gaz (315) accouplé à l'ouverture d'entrée du corps (303) en vue d'un mouvement orientable libre, le tube (315) mesurant moins de 200 mm de long ; et/ou
une extension de couverture de bouche (1203) comportant une sortie (1304) directement vers la bouche de l'utilisateur, de sorte que l'interface de patient (101) permette, lors de l'utilisation, d'administrer simultanément des gaz dans la bouche du patient par la sortie (1304) et dans des passages nasaux par le joint nasal (301).
